# EUROPEAN PATENT SPECIFICATION

(11) **EP 4 527 448 B1**
(45) Date of publication and mention of the grant of the patent: **02.07.2025**
(21) Application number: 24196107.7
(22) Date of filing: 23.08.2024
(51) Int. Cl.: A61N 1/368, A61N 1/372

(54) **DUAL CHAMBER LEADLESS PACEMAKER SYSTEMS THAT MONITOR AND ADJUST AV SYNCHRONY**
ZWEIKAMMER-HERZSCHRITTMACHERSYSTEME ZUR ÜBERWACHUNG UND ANPASSUNG DER AV-SYNCHRONITÄT
SYSTÈMES DE STIMULATEUR CARDIAQUE SANS FIL À DOUBLE CHAMBRE QUI SURVEILLENT ET AJUSTENT LA SYNCHRONIE AV

(30) Priority: 22.09.2023 US 202363584732 P; 15.08.2024 US 202418805834
(43) Date of publication of application: 26.03.2025
(73) Proprietor: Pacesetter, Inc., Sylmar, CA 91342 (US)
(72) Inventor: Goil, Aditya, Sylmar, 91342 (US); Badie, Nima, Sylmar, 91342 (US); Booth, Daniel F, Sylmar, 91342 (US)
(74) Representative: Barker Brettell Sweden AB

(56) References cited:
- US-A1- 2011 230 927
- US-A1- 2019 232 065
- US-A1- 2022 143 409
- US-B2- 10 987 518
- US-B2- 11 239 928

## Description

### FIELD OF TECHNOLOGY

Embodiments described herein generally relate to systems for monitoring and potentially adjusting (e.g., improving) atrioventricular (AV) synchrony provided by a dual chamber leadless pacemaker (LP) system.

### BACKGROUND

In certain dual chamber cardiac pacing systems, multiple leadless pacemakers wirelessly communicate with one another to reliably and safely coordinate pacing and/or sensing operations. Such a system may include, for example, a ventricular leadless pacemaker (vLP) implanted in or on a right ventricle (RV) and an atrial leadless pacemaker (aLP) implanted in or on the right atrium (RA), wherein the vLP and the aLP wirelessly communicate with one another to coordinate pacing and/or sensing operations. Such wireless communication between two IMDs (e.g., two LPs) is often referred to as implant-to-implant (i2i) communication. When using a pair of LPs to perform pacing and/or sensing operations in the RA and RV, one of the challenges is that bidirectional i2i communication is relied upon to maintain appropriate atrioventricular (AV) synchrony. However, for various different reasons, there may be periods of time during which bidirectional i2i communication fails, due to noise and/or the relative orientations of the LPs, but not limited thereto.

US 11,239,928 B2 discloses an IMD that wirelessly communicates another IMD, and methods for use therewith. Such a method can include receiving one or more i2i communication signals from the other IMD using a communication receiver of the IMD, measuring a strength of at least one of the one or more received i2i communication signals or a surrogate thereof, and updating a strength metric based on the measured strength or surrogate thereof. The method further includes determining, based on the updated strength metric, whether to increase, decrease, or maintain the sensitivity of the communication receiver of the IMD, and responding accordingly such that the sensitivity is sometimes increased, sometimes decreased, and sometimes maintained. The method can also include selectively causing a transmitter of the IMD to transmit an i2i communication signal to the other IMD requesting that the other IMD adjust its transmission strength.

US 2022/143409 A1 discloses a medical device configured to sense a cardiac signal that includes far field ventricular event signals and determine a ventricular activity metric from the sensed cardiac signal. The ventricular activity metric may be representative of a ventricular rate or an atrioventricular time interval. The medical device is configured to determine an atrioventricular synchrony metric based on the ventricular activity metric and generate an output based on the atrioventricular synchrony metric. The device may include a memory configured to store data corresponding to the atrioventricular synchrony metric.

US 10,987,518 B2 discloses an implantable system including an aLP and a vLP, and methods for use therewith, are configured or used to terminate a pacemaker mediated tachycardia (PMT). One of the aLP or the vLP detects a PMT and informs the other one. The aLP initiates a PMT PA interval that is shorter than a PA interval that the aLP would otherwise use for atrial pacing if a PMT was not detected. The vLP initiates a PMT PV interval that is longer than the PMT PA interval. If an intrinsic atrial or ventricular event is detected before PMT PA interval or the PMT PV interval expires, then these intervals will be terminated, otherwise an atrial chamber will be paced if the PMT PA interval expires, and/or a ventricular chamber will be paced if the PMT PV interval expires. This should have the effect of terminating the PMT.

US 2019/232065 A1 discloses systems and methods for monitoring chronic over-pacing (COP) to the heart are discussed herein. The system includes a receiver circuit to receive information about pacing rates of a plurality of paced heart beats, and a pacing analyzer circuit to generate a pacing rate distribution using pacing rates of the plurality of the paced heart beats. The pacing rate distribution includes a pacing rate histogram. The pacing analyzer circuit may recognize a morphological pattern from the pacing rate distribution, and detect a COP indication using the extracted feature. A programmer circuit adjusts one or more therapy parameters in response to the detected. COP indication.

US 2011/230927 A1 discloses an IMD for detecting transportless ventricular rhythm of a heart lacking atrial transport and comprises a housing, sensors configured to be located proximate to a heart, a sensing module to sense cardiac signals representative of a rhythm originating from the heart and a rhythm detection module. The rhythm detection module determines a change in AV association and identifies a potential ventricular complex with loss of atrial transport (VCLAT) based on the change in AV association.

### SUMMARY

The present invention is defined in the independent claim. Further embodiments of the invention are defined in the dependent claims.

Certain embodiments of the present technology are directed to a system including, or for use with, an atrial leadless pacemaker (aLP) and a ventricular leadless pacemaker (vLP) that are configured to communicate with one another, wherein the aLP and the vLP are configured to collectively provide DDD operation when an atrial-to-ventricular (a2v) message transmitted by the aLP is successfully received by the vLP, and a ventricular-to-atrial (v2a) message transmitted by the vLP is successfully received by the aLP, during a cardiac cycle. The aLP and the vLP are configured to collectively provide at least one of VDD operation, DDI operation or VDI operation at least some times when at least one of an a2v message transmitted by the aLP is not successfully received by the vLP, and/or a v2a message transmitted by the vLP is not successfully received by the aLP, during a further cardiac cycle. The system comprises one or more processors configured to obtain a2v throughput, v2a throughput, and event sequence information, for a period of time during which the aLP and the vLP were configured to collectively provide the DDD operation. The one or more processors of the system is/are also configured to determine, based on the a2v throughput and the v2a throughput, an estimated DDD metric, an estimated VDD metric, an estimated DDI metric, and an estimated VDI metric, for the period of time. The one or more processors of the system is/are also configured to determine an atrio-ventricular (AV) synchrony metric for the period of time based on the estimated DDD metric, the estimated VDD metric, the estimated DDI metric, the estimated VDI metric, and the event sequence information, wherein the AV synchrony metric specifies an estimate of how often AV synchrony was achieved during the period of time.

In accordance with certain embodiments, the system includes the aLP and the vLP, each of which includes a respective processor and a respective at least two electrodes, wherein the aLP is configured to be implanted in or on an atrial cardiac chamber and the vLP is configured to be implanted in or on a ventricular cardiac chamber. Additionally, the aLP is configured to transmit a2v messages to the vLP, the vLP is configured to attempt to receive the a2v messages from the aLP. The vLP is configured to transmit v2a messages to the aLP, and the aLP is configured to attempt to receive the v2a messages from the vLP.

In accordance with certain embodiments, the processor of the vLP or the processor of the aLP comprises at least one of the one or more processors configured to determine the AV synchrony metric. Additionally, at least one of the processor of the vLP or the processor of the aLP is further configured to adjust one or more parameters of at least one of the aLP or the vLP based on the AV synchrony metric.

In accordance with certain embodiments, the system includes a non-implanted subsystem (e.g., device) that includes at least one processor, of the one or more processors, that is/are configured to determine the AV synchrony metric for the period of time. The at least one processor of the non-implanted subsystem (e.g., device) is configured to adjust one or more parameters of at least one of the aLP or the vLP based on the AV synchrony metric.

In accordance with certain embodiments, the aLP and the vLP are configured to communicate with the non-implanted subsystem using conducted communication.

In accordance with certain embodiments, the non-implanted subsystem comprises a programmer that is configured to communicate with the aLP and the vLP.

In accordance with certain embodiments, the non-implanted subsystem comprises one of a tablet device, a smart phone, or a smart watch, that is configured to communicate with the aLP and the vLP.

In accordance with certain embodiments, the non-implanted subsystem comprises one of a bedside monitor, a patient link module, or a remote patient care network.

In accordance with certain embodiments, the one or more processors that is/are configured to determine the AV synchrony metric for the period of time, is/are also configured to adjust, based on the AV synchrony metric, at least one parameter used by the aLP for transmitting further a2v messages to the vLP, at least one parameter used by the vLP for receiving the further a2v messages from the aLP, at least one parameter used by the vLP for transmitting further v2a messages to the aLP, and/or at least one parameter used by the aLP for receiving the further v2a messages from the vLP.

In accordance with certain embodiments, the one or more processors that is/are configured to determine the AV synchrony metric for the period of time, is/are also configured to cause the AV synchrony metric to be displayed.

In accordance with certain embodiments, the one or more processors that is/are configured to determine the AV synchrony metric for the period of time, is/are also configured to provide a notification that an implant location of at least one of the aLP or the vLP should be modified.

In accordance with certain embodiments, the estimated DDD metric specifies an estimate of how often the aLP and the vLP collectively provided the DDD operation during the period of time during which the aLP and the vLP were configured to collectively provide the DDD operation. The estimated VDD metric specifies an estimate of how often the aLP and the vLP collectively provided the VDD operation during the period of time. The estimated DDI metric specifies an estimate of how often the aLP and the vLP collectively provided the DDI operation during the period of time. The estimated VDI metric specifies an estimate of how the aLP and the vLP collectively provided the VDI operation during the period of time.In accordance with certain embodiments, the event sequence information includes AP-VP information, AP-VS information, AS-VP information, and AS-VS information. The AP-VP information specifies how often the aLP provided atrial pacing (AP) and the vLP provided ventricular pacing (VP) for a same cardiac cycle within the period of time during which the aLP and the vLP were configured to collectively provide the DDD operation. The AP-VS information specifies how often the aLP provided AP and the vLP provided ventricular sensing (VS) for a same cardiac cycle within the period of time. The AS-VP information specifies how often the aLP provided atrial sensing (AS) and the vLP provided VP for a same cardiac cycle within the period of time. The AS-VS information specifies how often the aLP provided AS and the vLP provided VS for a same cardiac cycle within the period of time.

In accordance with certain embodiments, the aLP and the vLP are configured to communicate with one another using conducted communication.

Certain embodiments of the present technology are directed to a system including or for use with an atrial leadless pacemaker (aLP) and a ventricular leadless pacemaker (vLP) that are configured to communicate with one another and collectively provide DDD operation when an atrial-to-ventricular (a2v) message transmitted by the aLP is successfully received by the vLP and a ventricular-to-atrial (v2a) message transmitted by the vLP is successfully received by the aLP during a cardiac cycle, and wherein the aLP and the vLP are configured to collectively provide at least one of VDD, DDI or VDI operation at least some times when at least one of an a2v message transmitted by the aLP is not successfully received by the vLP and/or a v2a message transmitted by the vLP is not successfully received by the aLP during a further cardiac cycle, the system comprising one or more processors configured to: obtain a2v throughput, v2a throughput, and event sequence information, for a period of time during which the aLP and the vLP were configured to collectively provide DDD operation; determine, based on the a2v throughput and the v2a throughput, estimates of how often the aLP and the vLP collectively provide each of DDD, VDD, DDI, and VVI operation during the period of time; determine an atrio-ventricular (AV) synchrony metric for the period of time based on the estimates; and adjust, based on the AV synchrony metric, at least one parameter used by the aLP for communicating with the vLP and/or at least one parameter used by the vLP for communicating with the aLP. In accordance with certain embodiments, the system includes the aLP and the vLP, each of which includes a respective processor and a respective at least two electrodes; at least one of the processor of the vLP or the processor of the aLP comprises at least one of the one or more processors configured to determine the AV synchrony metric; and at least one of the processor of the vLP or the processor of the aLP is configured to adjust at least one parameter used by the aLP for communicating with the vLP and/or at least one parameter used by the vLP for communicating with the aLP. In accordance with other embodiments, the system includes a non-implanted subsystem that includes at least one processor, of the one or more processors, that is/are configured to determine the AV synchrony metric for the period of time and to adjust, based on the AV synchrony metric, at least one parameter used by the aLP for communicating with the vLP and/or at least one parameter used by the vLP for communicating with the aLP.

This summary is not intended to be a complete description of the embodiments of the present technology. Other features and advantages of the embodiments of the present technology will appear from the following description in which the preferred embodiments have been set forth in detail, in conjunction with the accompanying drawings and claims.

### BRIEF DESCRIPTION OF THE DRAWINGS

Embodiments of the present technology relating to both structure and method of operation may best be understood by referring to the following description and accompanying drawings, in which similar reference characters denote similar elements throughout the several views:
FIG. 1A illustrates a system formed in accordance with certain embodiments described herein as implanted in a heart.
FIG. 1B is a block diagram of an LP in accordance with certain embodiments herein.
FIG. 2 illustrates an LP in accordance with certain embodiments herein.
FIG. 3 is a timing diagram demonstrating one embodiment of conducted i2i communication for a paced event.
FIG. 4 is a timing diagram demonstrating one embodiment of conducted i2i communication for a sensed event.
FIG. 5 is a diagram that is used to show how the orientation of two different LPs can affect i2i communication, and thereby, a2v throughput and/or v2a throughput.
FIGS. 6A-6C are high level flow diagrams that are used to summarize certain embodiments of the present technology that determine and use an AV synchrony metric.
FIG. 7 is an example graph showing possible values (and more specifically, percentages) for a2v throughput and v2a throughput achieved by a dual chamber LP system.
FIG. 8 is an example graph showing how the a2v throughput and v2a throughput, introduced in FIG. 7, can be used to determine a DDDmax duration metric, and corresponding VDI, DDI, and VDD duration metrics.
FIG. 9 is an example graph showing how the a2v throughput and v2a throughput, introduced in FIG. 7, can be used to determine a DDDmin duration metric, and corresponding VDI, DDI, and VDD duration metrics.
FIG. 10 is a schematic that corresponds to the a2v throughput and v2a throughput, introduced in FIG. 7, and helps visualize the temporal overlap options for successful a2v communication and successful v2a communication.
FIGS. 11 and 12 are graphical representations of example distributions of event sequence information.
FIG. 13 shows a block diagram of one embodiment of an LP that is implanted into a patient as part of dual chamber LP system in accordance with certain embodiments herein.
FIG. 14 illustrates an example system that can be used to determine, display and otherwise use on or more AV synchrony metrics, in accordance with embodiments of the present technology.
FIG. 15 illustrates a system level diagram indicating potential devices and networks that utilize the methods and systems herein.

### DETAILED DESCRIPTION

A dual chamber implantable leadless pacemaker (LP) system in accordance with certain embodiments of the present technology includes an atrial LP (aLP) that is configured to be implanted in or the right atrium (RA) of a patient's heart and a ventricular LP (vLP) that is configured to be implanted in or on the right ventricle (RV) of the patient's heart. In certain embodiments, the aLP and the vLP are configured to provide bi-directional implant-to-implant (i2i) communication with one another to thereby enable them to collectively provide synchronized dual chamber cardiac pacing. More specifically, the aLP is configured to transmit atrial-to-ventricular (a2v) messages to the vLP, and the vLP is configured to attempt to receive the a2v messages from the aLP. The vLP is configured to transmit ventricular-to-atrial (v2a) messages to the aLP, and the aLP is configured to attempt to receive the v2a messages from the vLP. Such an a2v message, which is transmitted by the aLP and intended for reception by the vLP, can be used to inform the vLP of an intrinsic atrial event sensed by the aLP or a paced atrial event caused (or about to be caused) by the aLP. Such a v2a message, which is transmitted by the vLP and intended for reception by the aLP, can be used to inform the aLP of an intrinsic ventricular event sensed by the vLP or a paced ventricular event caused (or about to be caused) by the vLP. It is noted that the terms right ventricle (RV) and right ventricular (RV) chamber are used interchangeably herein, and that the acronym RV can stand for right ventricular or right ventricle, depending on the context. Similarly, the term right atrium (RA) and right atrial (RA) chamber are used interchangeably herein, and the acronym RA can stand for right atrial or right atrium, depending on the context.

When referring to various types of operation schemes (aka modes) herein, three letters are typically used to refer to the type of operation. In other words, a three position pacemaker code is often used, with the following nomenclature followed: the first position refers to the cardiac chamber paced; the second position refers to the cardiac chamber sensed; and the third position refers to the response to a sensed event. In the first and second positions, the letter O means none, the letter A means Atrium, the letter V means Ventricle, and the letter D means Dual (i.e., A and V). In the third position the letter O means none, the letter I means Inhibited, the letter T means Triggered (aka Tracked), and the letter D means Dual (i.e., T + I). The below Table 1 summarizes this pacemaker nomenclature. Where an R is included in a fourth position, that means the pacing that is provided is rate responsive.

**Table 1**

| Position 1 | Position 2 | Position 3 |
|---|---|---|
| (Chamber Paced) | (Chamber Sensed) | (Response to Sensed Event) |
| O = none | O = none | O = none |
| A = Atrium | A = Atrium | I = Inhibited |
| V = Ventricle | V = Ventricle | T = Triggered (aka Tracked) |
| D = Dual (A + V) | D = Dual (A + V) | D = Dual (I + T) |

DDD and DDD(R) operation modes, as is known in the art, provides for synchronized dual chamber pacing. More specifically, DDD operation provides for atrial and ventricular pacing, atrial and ventricular sensing, and the ability to both inhibit or trigger pacing following a sensed event. DDD(R) operation is DDD operation that is rate responsive, i.e., the pacing rate is adjusted based on the patient's activity level, e.g., as detected using a motion sensor and/or a temperature sensor. The term DDD operation, as used herein, also encompasses DDD(R) operation.

While neither the aLP nor the vLP can individually perform DDD operation, the aLP and the vLP can be configured to collectively provide for DDD operation (including DDD(R) operation) when there is successful bidirectional i2i communication, i.e., when the vLP successfully receives a2v messages from the aLP, and the aLP successfully receives v2a messages from the vLP.

However, for various different reasons, there may be periods of time during which bidirectional i2i communication fails, because the vLP fails to successfully receive one or more a2v messages from the aLP, and/or the aLP fails to successfully receive one or more v2a messages from the vLP. Such failures in i2i communication may occur, e.g., due to noise and/or the relative orientations of the aLP and vLP, but not limited thereto.

A dual chamber LP system may have safeguards in place to reduce asynchronous pacing in the event of loss of i2i communication in one or both directions, as outlined in the Table 2 below.

**Table 2**

| **Programmed mode** | **Automatic safeguard mode** | | |
|---|---|---|---|
| | **a2v comm. loss** | **v2a comm. loss** | **a2v and v2a (i.e., bi-directional) comm. loss** |
| DDD | DDI | VDD | VDI |
| DDI | DDI | VDI | VDI |
| VDD | VDI | VDD | VDI |
| DOO | DOO | VOO | VOO |

As can be appreciated from Table 2 above, in a dual chamber LP system including an aLP and vLP that are configured to collectively provide DDD operation, if a2v communication is lost (i.e., one or more a2v messages transmitted by the aLP are not successfully received by the vLP), then the vLP stops tracking atrial activity, and the dual chamber LP system effectively provides DDI operation. If v2a communication is lost (i.e., one or more v2a messages transmitted by the vLP are not successfully received by the aLP), then the aLP withholds pacing, and the dual chamber LP system effectively provides VDD operation. If i2i communication is simultaneously lost in both directions (i.e., v2a messages transmitted by the vLP are not successfully received by the aLP, and a2v messages transmitted by the aLP are not successfully received by the vLP), both mitigations take effect, and the dual chamber LP system effectively provides VDI operation. These transmission receipt safeguards act to guarantee RV pacing while maintaining RA tracking and RA pacing whenever possible.

The vLP is configured to transmit an acknowledgement (ACK) message when the vLP successfully receives an a2v message from the aLP, and the aLP is configured to transmit an ACK message to the vLP when the aLP successfully receives a v2a message from the vLP. In this manner, the aLP can keep track of its a2v throughput, and the vLP can keep track of its v2a throughput, both of which can be uploaded from one or both the LPs to an external system (e.g., an external programmer, patient monitor, or patient link module) that communicates with one or both of the LPs. For example, the aLP can transit a2v throughput information to an external system, and the vLP can separate transmit v2a throughput information to the external system. For another example, the aLP can transmit a2v throughput information to the vLP, and the vLP can transmit both v2a throughput information and a2v throughput information to the external system. For the purpose of this discussion, the aLP is considered to have successfully received a v2a message when the aLP successfully detects and decodes the v2a message, and the vLP is considered to have successfully received an a2v message when the vLP successfully detects and decodes the v2a message.

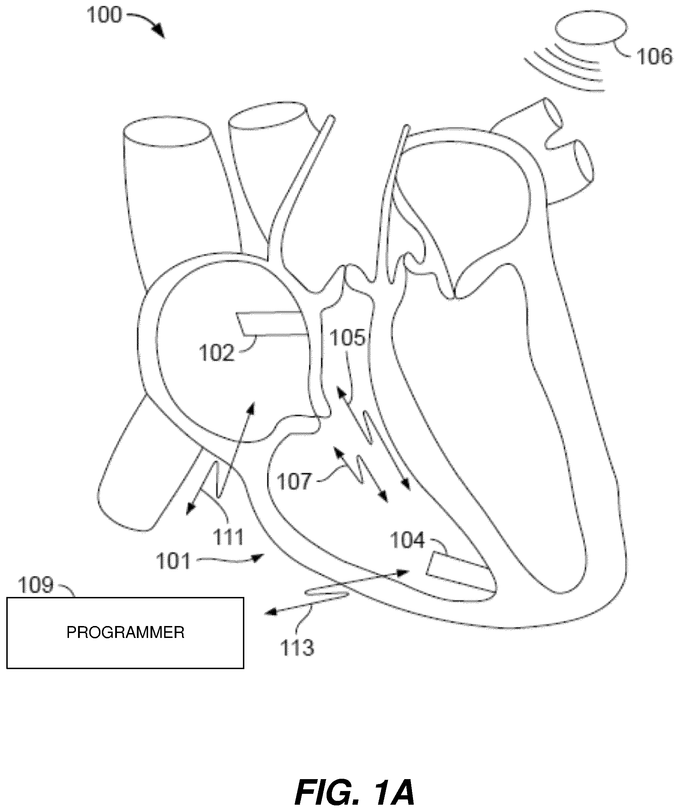

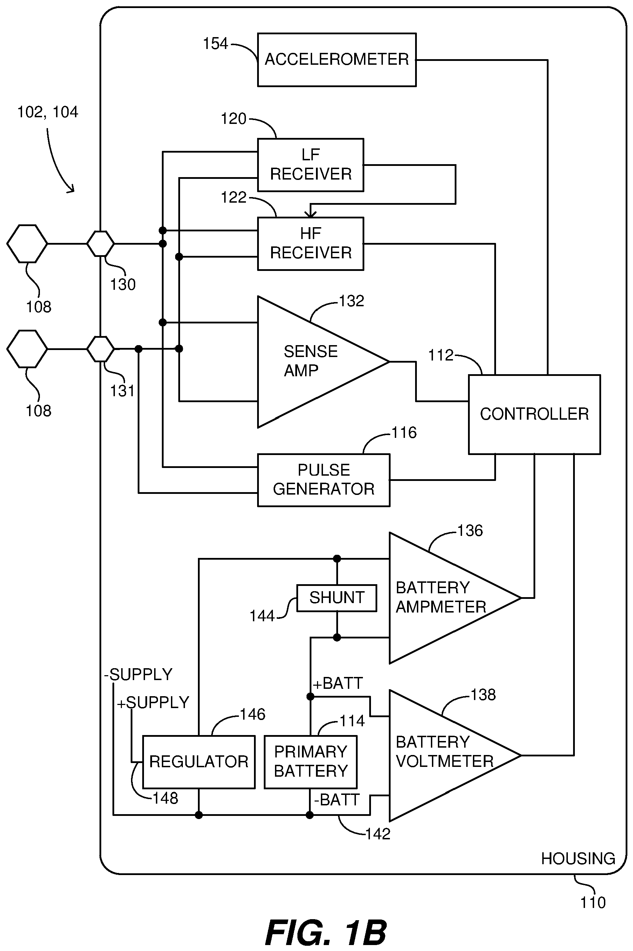

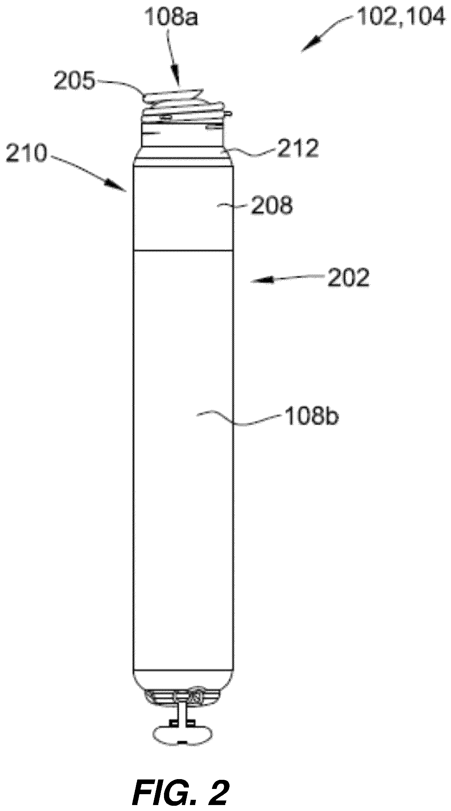

Once the a2v throughput and v2a throughput information is provided to an external system (e.g., an external programmer, or a patient care network (PCN)), a user (e.g., clinician and/or physician) of the external system may be presented (e.g., via a display monitor) with the throughput information. For example, the a2v throughput information may specify the percentage of a2v messages transmitted by the aLP that were successfully received by the vLP, and the v2a throughput information may specify the percentage of v2a messages transmitted by the vLP that were successfully received by the aLP. However, such a2v and v2a throughput information on its own does not provide the user with an indication of the percentage of cardiac cycles during which DDD operation was effectively achieved, and also does not provide the user with an indication of the percentage of cardiac cycles during which AV synchrony was achieved. Certain embodiments of the present technology described herein compute an estimate of how often a dual chamber LP system (including an aLP and a vLP) provided DDD operation, as well as in one or more safeguard types of pacing. Additionally, certain embodiments of the present technology described herein compute an estimate of how often the dual chamber LP system provided AV synchrony. Further, in accordance with certain embodiments of the present technology described herein, at least one parameter used by the aLP for transmitting further a2v messages to the vLP, at least one parameter used by the vLP for receiving the further a2v messages from the aLP, at least one parameter used by the vLP for transmitting further v2a messages to the aLP, and/or at least one parameter used by the aLP for receiving the further v2a messages from the vLP is adjusted based on the estimated AV synchrony. Additional details of such embodiments are provided herein. However, before providing addition details of the specific embodiments of the present technology mentioned above, an exemplary system in or with which embodiments of the present technology can be used will first be described with reference to FIGS. 1A, 1B and 2.

FIG. 1A illustrates a system 100 formed in accordance with certain embodiments herein as implanted in a heart 101. The system 100 comprises two LPs 102 and 104 located in different chambers of the heart. The LP 102 is located in a right atrium (RA), while the LP 104 is located in a right ventricle (RV). Accordingly, the LP 102 can also be referred to more specifically as the aLP 102, and the LP 104 can also be referred to more specifically as the vLP 104. The RA is also known as the right atrial chamber, and the RV is also known as the right ventricular chamber. Such chambers of a patient's heart can also be referred to herein as cardiac chambers. LPs 102 and 104 communicate with one another to inform one another of various local physiologic activities, such as local intrinsic events, local paced events, and the like. LPs 102 and 104 may be constructed in a similar manner, but operate differently based upon which chamber LP 102 or 104 is located.

In some embodiments, LPs 102 and 104 communicate with one another, as well as with an optional ICD 106, and with an external system (e.g., programmer) 109 through wireless transceivers, communication coils and antenna, and/or by conducted i2i communication through the same electrodes as used for sensing and/or delivery of pacing therapy. Hence, in an embodiment, the LPs 102 and 104 are configured to communicate with one another using conducted communication. When conducted i2i communication is maintained through the same electrodes as used for pacing, the system 100 may omit an antenna or telemetry coil in one or more of LPs 102 and 104. For most of the following discussion, it is assumed that the LPs 102 and 104 communicate with one another, as well as other devices, using conducted communication, thereby eliminating the need for the LPs 102 and 104 to include an antenna or a telemetry coil. Nevertheless, it is noted that many of the embodiments described herein can also be used where the LPs 102 and 104 utilize antennas and/or telemetry coils for performing i2i communication. Further, it is also noted that conducted communication can also be equivalently referred to as conductive communication.

In an embodiment, the LPs 102 and 104 are configured to communicate with the non-implanted subsystem 109 using conducted communication.

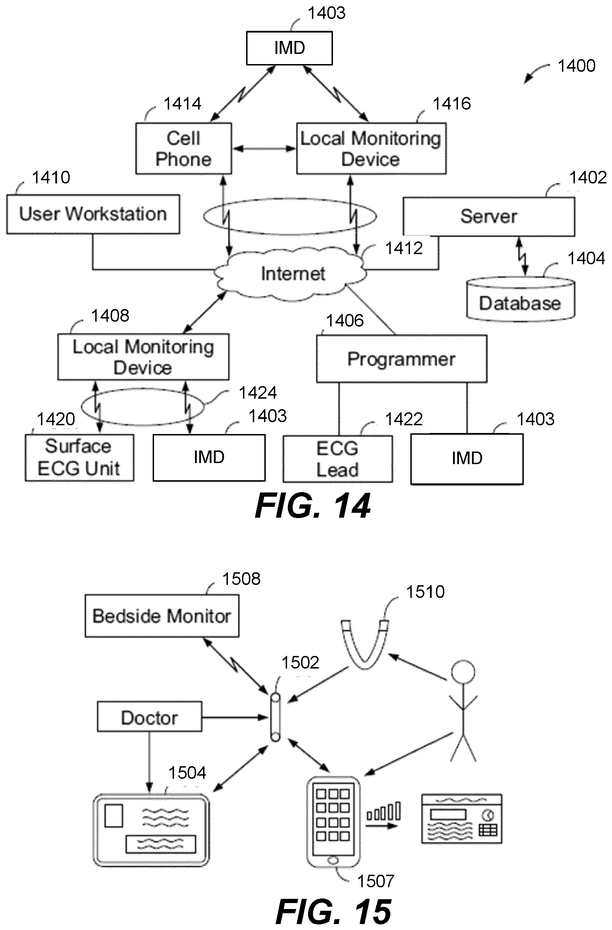

The non-implanted subsystem 109 may, for instance, comprise a programmer 1406 that is configured to communicate with the LPs 102 and 104 as shown in Fig. 14. In further embodiments, the non-implanted subsystem 109 may comprise a tablet device 1504, a smart phone 1506 and/or a smart watch that is configured to communicate with the LPs 102 and 104 as shown in Fig. 15. In certain embodiments, the non-implanted subsystem 109 comprises a bedside monitor 1508, a patient link module and/or a remote patient care network as shown in Fig. 15.

In some embodiments, one or more LPs 102 and 104 can be co-implanted with the ICD 106, however that need not be the case. Each LP 102, 104 uses two or more electrodes located within, on, or within a few centimeters of the housing of the LP, for pacing and sensing at the cardiac chamber, for bidirectional communication with one another, with an external system (e.g., programmer) 109, and the ICD 106.

Referring to FIG. 1B, a block diagram shows exemplary electronics within LPs 102 and 104. LP 102, 104 includes first and second receivers 120 and 122 that collectively define separate first and second communication channels 105 and 107 (FIG. 1A), (among other things) between LPs 102 and 104. Although first and second receivers 120 and 122 are depicted, in other embodiments, LP 102, 104 may only include first receiver 120, or may include additional receivers other than first and second receivers 120 and 122. As will be described in additional detail below, the pulse generator 116 can function as a transmitter that transmits i2i communication signals using the electrodes 108. Usage of the electrodes 108 for communication enables the one or more LPs 102 and 104 to perform antenna-less and telemetry coil-less communication. A same pulse generator 116 can be used to produce both pacing pulses and conducted communication pulses, in which case an LP may only include a single pulse generator 116. Alternatively, an LP can include more than one pulse generator, e.g., one of which can be used to produce pacing pulses, and the other can be used to produce conducted communication pulses.

In accordance with certain embodiments, when one of the LPs 102 and 104 senses an intrinsic event or delivers a paced event, the corresponding LP 102, 104 transmits an implant event message to the other LP 102, 104. For example, when an aLP 102 senses/paces an atrial event, the aLP 102 transmits an implant event message including an event marker indicative of a nature of the event (e.g., intrinsic/sensed atrial event, paced atrial event). When a vLP 104 senses/paces a ventricular event, the vLP 104 transmits an implant event message including an event marker indicative of a nature of the event (e.g., intrinsic/sensed ventricular event, paced ventricular event). In certain embodiments, LP 102, 104 transmits an implant event message to the other LP 102, 104 preceding the actual pace pulse so that the remote LP can blank its sense inputs in anticipation of that remote pace pulse (to prevent inappropriate crosstalk sensing).

Still referring to FIG. 1B, each LP 102, 104 is shown as including a controller 112 and a pulse generator 116. The controller 112 can include, e.g., a microprocessor (or equivalent control circuitry), RAM and/or ROM memory, logic and timing circuitry, state machine circuitry, and I/O circuitry, but is not limited thereto. The controller 112 can further include, e.g., timing control circuitry to control the timing of the stimulation pulses (e.g., pacing rate, atrio-ventricular (AV) delay, atrial interconduction (A-A) delay, or ventricular interconduction (V-V) delay, etc.). Such timing control circuitry may also be used for the timing of refractory periods, blanking intervals, noise detection windows, evoked response windows, alert intervals, marker channel timing, and so on. The controller 112 can further include other dedicated circuitry and/or firmware/software components that assist in monitoring various conditions of the patient's heart and managing pacing therapies. The controller 112 and the pulse generator 116 may be configured to transmit event messages, via the electrodes 108, in a manner that does not inadvertently capture the heart in the chamber where LP 102, 104 is located, such as when the associated chamber is in a refractory state. In addition, an LP 102, 104 that receives an event message may enter an "event refractory" state (or event blanking state) following receipt of the event message. The event refractory/blanking state may be set to extend for a determined period of time after receipt of an event message in order to avoid the receiving LP 102, 104 from inadvertently sensing another signal as an event message that might otherwise cause retriggering. For example, the receiving LP 102, 104 may detect a measurement pulse from another LP 102, 104 or programmer 109.

In accordance with certain embodiments herein, the programmer 109 may communicate over a programmer-to-LP channel, with LP 102, 104 utilizing the same communication scheme. The external programmer 109 may listen to the event message transmitted between LP 102, 104 and synchronize programmer to implant communication such that programmer 109 does not transmit communication signals 113 until after an implant to implant messaging sequence is completed.

In some embodiments, the individual LP 102 (or 104) can comprise a hermetic housing 110 configured for placement on or attachment to the inside or outside of a cardiac chamber and at least two leadless electrodes 108 proximal to the housing 110 and configured for bidirectional communication with at least one other device (e.g., a co-implanted ICD 106) within or outside the body.

Referring to FIG. 1B, the LP 102 (or 104) is shown as optionally including an accelerometer 154 which can be hermetically contained within the housing 110. The accelerometer 154 can be any one of various different types of well-known accelerometers, or can be a future developed accelerometer. For one example, the accelerometer 154 can be or include, e.g., a MEMS (micro-electromechanical system) multi-axis accelerometer of the type exploiting capacitive or optical cantilever beam techniques, or a piezoelectric accelerometer that employs the piezoelectric effect of certain materials to measure dynamic changes in mechanical variables. Where the accelerometer is a multi-axis accelerometer it can include two or three sensors aligned along orthogonal axes. The accelerometer 154 can be, e.g., a one-dimensional (1D) accelerometer (also known as a one-axis accelerometer), a two-dimensional (2D) accelerometer (also known as a two-axis accelerometer), or a three-dimensional (3D) accelerometer (also known as a three-axis accelerometer). The output(s) of the accelerometer can be used to determine the orientation of the IMD, and thus, it can be said that the output(s) of the accelerometer (e.g., 154) are indicative of an orientation of the IMD (e.g., LP 102 or 104). More specifically, in accordance with certain embodiments, the controller 112 of an LP 102 (or 104) receives one or more outputs output(s) of the accelerometer 154, which is/are indicative of an orientation of the LP 102 (or 104). In such embodiments, the controller 112 can determine, based on the output(s) received from the accelerometer 154, an actual orientation of the LP 102 (or 104). Each output of the accelerometer 154 can comprise a respective signal.

One or more signals produced and output by the accelerometer 154 may be analyzed with respect to frequency content, energy, duration, amplitude and/or other characteristics. Such signals may or may not be amplified and/or filtered prior to being analyzed. For example, filtering may be provided using lowpass, highpass and/or bandpass filters. The signals output by the accelerometer 154 can be analog signals, which can be analyzed in the analog domain, or can be converted to digital signals (by an analog-to-digital converter) and analyzed in the digital domain. Alternatively, the signals output by the accelerometer 154 can already be in the digital domain. The one or more signals output by the accelerometer 154 can be analyzed by the controller 112 and/or other circuitry. In certain embodiments, the accelerometer 154 is packaged along with an integrated circuit (IC) that is designed to analyze the signal(s) it generates. In such embodiments, one or more outputs of the packaged sensor/IC can be an indication of acceleration along one or more axes. In other embodiments, the accelerometer 154 can be packaged along with an IC that performs signal conditioning (e.g., amplification and/or filtering), performs analog-to-digital conversions, and stores digital data (indicative of the sensor output) in memory (e.g., RAM, which may or may not be within the same package). In such embodiments, the controller 112 or other circuitry can read the digital data from the memory and analyze the digital data. Other variations are also possible, and within the scope of embodiments of the present technology. In accordance with certain embodiments, the LPs and/or other IMDs are devoid of an accelerometer 154.

FIG. 1B depicts a single LP 102 (or 104) and shows the LP's functional elements substantially enclosed in a hermetic housing 110. The LP 102 (or 104) has at least two electrodes 108 located within, on, or near the housing 110, for delivering pacing pulses to and sensing electrical activity from the muscle of the cardiac chamber, for sensing motion, for sensing temperature, and for bidirectional conducted communication with at least one other device within or outside the body. Hermetic feedthroughs 130, 131 conduct electrode signals through the housing 110. The housing 110 contains a primary battery 114 to supply power for pacing, sensing, and communication. The housing 110 also contains circuits 132 for sensing cardiac activity from the electrodes 108, receivers 120, 122 for receiving information from at least one other device via the electrodes 108, and the pulse generator 116 for generating pacing pulses for delivery via the electrodes 108 and also for transmitting information to at least one other device via the electrodes 108. The housing 110 can further contain circuits for monitoring device health, for example a battery current monitor 136 and a battery voltage monitor 138, and can contain circuits for controlling operations in a predetermined manner.

The receivers 120 and 122 can also be referred to, respectively, as a low frequency (LF) receiver 120 and a high frequency (HF) receiver 122, because the receiver 120 is configured to monitor for one or more signals within a relatively low frequency range (e.g., below 100 KHz) and the receiver 122 is configured to monitor for one or more signals within a relatively high frequency range (e.g., above 100 KHz). In certain embodiments, the receiver 120 (and more specifically, at least a portion thereof) is always enabled and monitoring for a wakeup notice, which can simply be a wakeup pulse, within a specific low frequency range (e.g., between 1 KHz and 100 KHz); and the receiver 122 is selectively enabled by the receiver 120. The receiver 120 is configured to consume less power than the receiver 122 when both the first and second receivers are enabled. Accordingly, the receiver 120 can also be referred to as a low power receiver 120, and the receiver 122 can also be referred to as a high power receiver 122. The low power receiver 120 is incapable of receiving signals within the relatively high frequency range (e.g., above 100 KHz), but consumes significantly less power than the high power receiver 122. This way the low power receiver 120 is capable of always monitoring for a wakeup notice without significantly depleting the battery (e.g., 114) of the LP. In accordance with certain embodiments, the high power receiver 122 is selectively enabled by the low power receiver 120, in response to the low power receiver 120 receiving a wakeup notice, so that the high power receiver 122 can receive the higher frequency signals, and thereby handle higher data throughput needed for effective i2i communication without unnecessarily and rapidly depleting the battery of the LP (which the high power receiver 122 may do if it were always enabled).

Since the receivers 120, 122 are used to receive conducted communication messages, the receivers 120, 122 can also be referred to as conducted communication receivers. In certain embodiments, each of the LPs 102 includes only a single conducted communication receiver. An example of a single conducted communication receiver, the can be included in the LPs and/or other types of IMDs referred to herein, is described in commonly assigned U.S. Patent Publication No. 2023/0171130 A1.

The electrodes 108 can be configured to communicate bidirectionally among the multiple LPs and/or the implanted ICD 106 to coordinate pacing pulse delivery and optionally other therapeutic or diagnostic features using messages that identify an event at an individual LP originating the message and an LP receiving the message react as directed by the message depending on the origin of the message. An LP 102, 104 that receives the event message reacts as directed by the event message depending on the message origin or location. In some embodiments or conditions, the two or more leadless electrodes 108 can be configured to communicate bidirectionally among the one or more LPs 102, 104 and/or the ICD 106 and transmit data including designated codes for events detected or created by an individual LP. Individual LPs can be configured to issue a unique code corresponding to an event type and a location of the sending pacemaker.

Moreover, information communicated on the incoming channel can also include an event message from another leadless cardiac pacemaker signifying that the other leadless cardiac pacemaker has sensed a heartbeat or has delivered a pacing pulse, and identifies the location of the other pacemaker. For example, LP 104 may receive and relay an event message from LP 102 to the programmer. Similarly, information communicated on the outgoing channel can also include a message to another LP, or to the ICD, that the sending leadless cardiac pacemaker has sensed a heartbeat or has delivered a pacing pulse at the location of the sending pacemaker.

Referring again to FIGS. 1 and 2, the cardiac pacing system 100 may comprise an ICD 106 in addition to LPs 102, 104 configured for implantation in electrical contact with a cardiac chamber and for performing cardiac rhythm management functions in combination with the implantable ICD 106. The implantable ICD 106 and the one or more LPs 102, 104 can be configured for leadless intercommunication by information conduction through body tissue for wireless transmission between transmitters and receivers in accordance with the discussion herein.

As shown in the illustrative embodiments, an LP 102, 104 can comprise two or more leadless electrodes 108 configured for delivering cardiac pacing pulses, sensing evoked and/or natural cardiac electrical signals, and bidirectionally communicating with the co-implanted ICD 106.

LP 102, 104 can be configured for operation in a particular location and a particular functionality at manufacture and/or at programming by an external programmer 109. Bidirectional communication among the multiple LPs can be arranged to communicate notification of a sensed heartbeat or delivered pacing pulse event and encoding type and location of the event to another implanted pacemaker or pacemakers. LP 102, 104 receiving the communication decode the information and respond depending on location of the receiving pacemaker and predetermined system functionality.

In some embodiments, the LPs 102 and 104 are configured to be implantable in or on any chamber of the heart, namely either atrium (RA, LA) or either ventricle (RV, LV). Furthermore, for dual-chamber configurations, multiple LPs may be co-implanted (e.g., one in the RA and one in the RV, or one in the RV and one in the coronary sinus proximate the LV). Certain pacemaker parameters and functions depend on (or assume) knowledge of the chamber in which the pacemaker is implanted (and thus with which the LP is interacting; e.g., pacing and/or sensing). Some non-limiting examples include: sensing sensitivity, an evoked response algorithm, use of AF suppression in a local chamber, blanking and refractory periods, etc. Accordingly, each LP preferably knows an identity of the chamber in which the LP is implanted, and processes may be implemented to automatically identify a local chamber associated with each LP.

Also shown in FIG. 1B, the primary battery 114 has positive terminal 140 and negative terminal 142. Current from the positive terminal 140 of primary battery 114 flows through a shunt 144 to a regulator circuit 146 to create a positive voltage supply 148 suitable for powering the remaining circuitry of the pacemaker 102. The shunt 144 enables the battery current monitor 136 to provide the controller 112 with an indication of battery current drain and indirectly of device health. The illustrative power supply can be a primary battery 114.

In various embodiments, LP 102, 104 can manage power consumption to draw limited power from the battery, thereby reducing device volume. Each circuit in the system can be designed to avoid large peak currents. For example, cardiac pacing can be achieved by discharging a tank capacitor (not shown) across the pacing electrodes. Recharging of the tank capacitor is typically controlled by a charge pump circuit. In a particular embodiment, the charge pump circuit is throttled to recharge the tank capacitor at constant power from the battery.

In some embodiments, the controller 112 in one LP 102, 104 can access signals on the electrodes 108 and can examine output pulse duration from another pacemaker for usage as a signature for determining triggering information validity and, for a signature arriving within predetermined limits, activating delivery of a pacing pulse following a predetermined delay of zero or more milliseconds. The predetermined delay can be preset at manufacture, programmed via an external programmer, or determined by adaptive monitoring to facilitate recognition of the triggering signal and discriminating the triggering signal from noise. In some embodiments or in some conditions, the controller 112 can examine output pulse waveform from another leadless cardiac pacemaker for usage as a signature for determining triggering information validity and, for a signature arriving within predetermined limits, activating delivery of a pacing pulse following a predetermined delay of zero or more milliseconds.

In certain embodiments, the electrodes of an LP 102, 104 can be used to sense an intracardiac electrocardiogram (IEGM) from which atrial and/or ventricular activity can be detected, e.g., by detecting QRS complexes and/or P waves. Such an IEGM can also be used by an LP 102, 104 to time when communication pulses should be generated, since the orientation of the LPs 102, 104 relative to one another, and the distances between the LPs 102, 104, can change throughout each cardiac cycle. In other words, an LP can utilize a sensed IEGM to determine timing relative to a cardiac cycle.

FIG. 2 shows an LP 102, 104. The LP can include a hermetic housing 202 (e.g., the housing 110 in FIG. 1) with electrodes 108a and108b disposed thereon. As shown, electrode 108a can be separated from but surrounded partially by a fixation mechanism 205, and the electrode 108b can be disposed on the housing 202. The fixation mechanism 205 can be a fixation helix, a plurality of hooks, barbs, or other attaching features configured to attach the pacemaker to tissue, such as heart tissue. The electrodes 108a and 108b are examples of the electrodes 108 shown in and discussed above with reference to FIG. 1B. One of the electrodes 108 (e.g., 108a) can function as a cathode type electrode and another one of the electrodes 108 (e.g., 108b) can function as an anode type electrode, or vice versa, when the electrodes are used for delivering stimulation. For the purpose of this discussion, the electrode 108a will often be referred to as the button electrode 108a (or the tip electrode 108a), and the electrode 108b will often be referred to as the ring electrode 108b.

The housing 202 can also include an electronics compartment 210 within the housing that contains the electronic components necessary for operation of the pacemaker, including, e.g., a pulse generator, receiver, a battery, and a processor for operation. The hermetic housing 202 can be adapted to be implanted on or in a human heart, and can be cylindrically shaped, rectangular, spherical, or any other appropriate shapes, for example.

The housing 202 can comprise a conductive, biocompatible, inert, and anodically safe material such as titanium, 316L stainless steel, or other similar materials. The housing 202 can further comprise an insulator disposed on the conductive material to separate electrodes 108a and 108b. The insulator can be an insulative coating on a portion of the housing between the electrodes, and can comprise materials such as silicone, polyurethane, parylene, or another biocompatible electrical insulator commonly used for implantable medical devices. In the embodiment of FIG. 2, a single insulator 208 is disposed along the portion of the housing between electrodes 108a and 108b. In some embodiments, the housing itself can comprise an insulator instead of a conductor, such as an alumina ceramic or other similar materials, and the electrodes can be disposed upon the housing.

As shown in FIG. 2, the pacemaker can further include a header assembly 212 to isolate the electrodes 108a and 108b. The header assembly 212 can be made from PEEK, tecothane or another biocompatible plastic, and can contain a ceramic to metal feedthrough, a glass to metal feedthrough, or other appropriate feedthrough insulator as known in the art.

The electrodes 108a and 108b can comprise pace/sense electrodes, or return electrodes. A low-polarization coating can be applied to the electrodes, such as sintered platinum, platinum-iridium, iridium, iridium-oxide, titanium-nitride, carbon, or other materials commonly used to reduce polarization effects, for example. In FIG. 2, electrode 108a can be a pace/sense electrode and electrode 108b can be a return electrode. The electrode 108b can be a portion of the conductive housing 202 that does not include an insulator 208.

Several techniques and structures can be used for attaching the housing 202 to the interior or exterior wall of the heart. A helical fixation mechanism 205, can enable insertion of the device endocardially or epicardially through a guiding catheter. A torqueable catheter can be used to rotate the housing and force the fixation device into heart tissue, thus affixing the fixation device (and also the electrode 108a in FIG. 2) into contact with stimulable tissue. Electrode 108b can serve as an indifferent electrode for sensing and pacing. The fixation mechanism may be coated partially or in full for electrical insulation, and a steroid-eluting matrix may be included on or near the device to minimize fibrotic reaction, as is known in conventional pacing electrode-leads.

### Implant-to-Implant Event Messaging

LPs 102 and 104 can utilize implant-to-implant (i2i) communication through event messages to coordinate operation with one another in various manners. The terms i2i communication, i2i event messages, and i2i even markers are used interchangeably herein to refer to event related messages and IMD/IMD operation related messages transmitted from an implanted device and directed to another implanted device (although external systems, e.g., a programmer, may also receive i2i event messages). In certain embodiments, LP 102 and LP 104 operate as two independent leadless pacers maintaining beat-to-beat dual-chamber functionality via a "Master/Slave" operational configuration. For descriptive purposes, the ventricular LP 104 shall often be referred to as "vLP" and the atrial LP 102 shall often be referred to as "aLP". The LP 102 or 104 that is designated as the master device (e.g. vLP) may implement all or most dual-chamber diagnostic and therapy determination algorithms. For purposes of the following illustration, it is assumed that the vLP is a "master" device, while the aLP is a "slave" device. Alternatively, the aLP may be designated as the master device, while the vLP may be designated as the slave device. The master device orchestrates most or all decision-making and timing determinations (including, for example, rate-response changes).

In accordance with certain embodiments, methods are provided for coordinating operation between first and second LPs configured to be implanted entirely within first and second chambers of the heart. It is alternatively possible that an LP is implanted on an exterior of a cardiac chamber, rather than within a cardiac chamber. In certain embodiments, a method transmits an event marker using conducted communication through electrodes located along a housing of the first LP, wherein the event marker is indicative of one of a local paced or sensed event. The method detects, over a sensing channel, the event marker at the second LP. The method identifies the event marker at the second LP based on a predetermined pattern configured to indicate that an event of interest has occurred in a remote chamber. In response to the identifying operation, the method initiates a related action in the second LP.

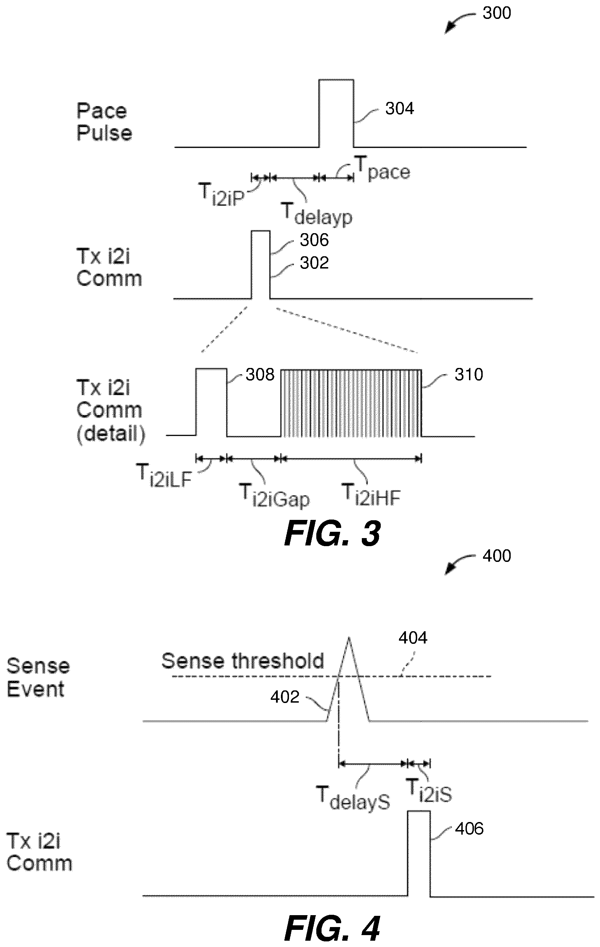

FIG. 3 is a timing diagram 300 demonstrating one example of a conducted i2i communication for a paced event. The i2i communication may be transmitted, for example, from LP 102 to LP 104. As shown in FIG. 3, in this embodiment, an i2i transmission 302 is sent prior to delivery of a pace pulse 304 by the transmitting LP (e.g., LP 102). This enables the receiving LP (e.g., LP 104) to prepare for the remote delivery of the pace pulse. The i2i transmission 302 includes an envelope 306 that may include one or more individual pulses. For example, in this embodiment, envelope 306 includes a low frequency pulse 308 followed by a high frequency pulse train 310. Low frequency pulse 308 lasts for a period T_{i2iLF}, and high frequency pulse train 310 lasts for a period T_{i2iHF}. The end of low frequency pulse 308 and the beginning of high frequency pulse train 310 are separated by a gap period, T_{i2iGap}.

As shown in FIG. 3, the i2i transmission 302 lasts for a period T_{i2iP}, and pace pulse 304 lasts for a period Tpace. The end of i2i transmission 302 and the beginning of pace pulse 304 are separated by a delay period, T_{delayp}. The delay period may be, for example, between approximately 0.0 and 10.0 milliseconds (msec), particularly between approximately 0.1 msec and 2.0 msec, and more particularly approximately 1.0 msec. The terms approximately and about, as used herein, mean +/- 10% of a specified value.

FIG. 4 is a timing diagram 400 demonstrating one example of an i2i communication for a sensed event. The i2i communication may be transmitted, for example, from LP 102 to LP 104. As shown in FIG. 4, in this embodiment, the transmitting LP (e.g., LP 102) detects the sensed event when a sensed intrinsic activation 402 crosses a sense threshold 404. A predetermined delay period, T_{delayS}, after the detection, the transmitting LP transmits an i2i transmission 406 that lasts a predetermined period T_{i2iS}. The delay period may be, for example, between approximately 0.0 and 10.0 milliseconds (msec), particularly between approximately 0.1 msec and 2.0 msec, and more particularly approximately 1.0 msec.

As with i2i transmission 302, i2i transmission 406 may include an envelope that may include one or more individual pulses. For example, similar to envelope 306, the envelope of i2i transmission 406 may include a low frequency pulse followed by a high frequency pulse train.

In accordance with certain embodiments, the duration of each of the periods T_{i2iP} and T_{i2iS} can be in the range of 1.5 msec to 13 msec, and more preferably, be within the range of 2.0 msec to 5.0 msec. This duration includes the both the low frequency pulse (e.g., 308) and the high frequency pulse train (e.g., 310, that follows the low frequency pulse). The low frequency pulse (e.g., 308) can be used as a wakeup pulse, and the high frequency pulse train (e.g., 310, that follows the low frequency pulse) can be used as a communication event marker and payload.

Where i2i communication is provided using conducted communication, it will often be referred to herein more specifically as conducted i2 communication, or equivalently i2i conducted communication. It is also noted that the phase conducted communication is often used interchangeably with the phase conductive communication.

Optionally, wherein the first LP is located in an atrium and the second LP is located in a ventricle, the first LP produces an AS/AP event marker to indicate that an atrial sensed (AS) event or atrial paced (AP) event has occurred or will occur in the immediate future. For example, the AS and AP event markers may be transmitted following the corresponding AS or AP event. Alternatively, the first LP may transmit the AP event marker slightly prior to delivering an atrial pacing pulse. Where the first LP is located in an atrium and the second LP is located in a ventricle, the second LP can initiate an atrioventricular (AV) interval after receiving an AS or AP event marker from the first LP; and can initiate a post atrial ventricular blanking (PAVB) interval after receiving an AP event marker from the first LP.

Optionally, the first and second LPs may operate in a "pure" master/slave relation, where the master LP delivers "command" markers in addition to or in place of "event" markers. A command marker directs the slave LP to perform an action such as to deliver a pacing pulse and the like. For example, when a slave LP is located in an atrium and a master LP is located in a ventricle, in a pure master/slave relation, the slave LP delivers an immediate pacing pulse to the atrium when receiving an AP command marker from the master LP.

In accordance with some embodiments, communication and synchronization between the aLP and vLP is implemented via conducted communication of markers/commands in the event messages (per an i2i communication protocol). In accordance with certain embodiments, conducted communication represents event messages transmitted from the sensing/pacing electrodes at frequencies outside the RF or Wi-Fi frequency range. Such conducted communication relies on electrical communication signals or pulses that "conducted" through a patient's body, and more specifically, through patient tissue. The figures and corresponding description below illustrate non-limiting examples of markers that may be transmitted in event messages. The figures and corresponding description below also include the description of the markers and examples of results that occur in the LP that receives the event message. Table 3 represents exemplary event markers sent from the aLP to the vLP, while Table 4 represents exemplary event markers sent from the vLP to the aLP. In the master/slave configuration, AS event markers are sent from the aLP each time that an atrial event is sensed outside of the post ventricular atrial blanking (PVAB) interval or some other alternatively-defined atrial blanking period. The AP event markers are sent from the aLP each time that the aLP delivers a pacing pulse in the atrium. The aLP may restrict transmission of AS markers, whereby the aLP transmits AS event markers when atrial events are sensed both outside of the PVAB interval and outside the post ventricular atrial refractory period (PVARP) or some other alternatively-defined atrial refractory period. Alternatively, the aLP may not restrict transmission of AS event markers based on the PVARP, but instead transmit the AS event marker every time an atrial event is sensed.

**Table 3**

| "A2V" Markers / Commands (*i.e.*, from aLP to vLP) | | |
|---|---|---|
| *Marker* | *Description* | *Result in vLP* |
| AS | Notification of a sensed event in atrium (if not in PVAB or PVARP) | • Initiate AV interval (if not in PVAB or PVARP) |
| AP | Notification of a paced event in atrium | • Initiate PAVB |
| | | • Initiate AV interval (if not in PVARP) |

As shown in Table 3, when an aLP transmits an event message that includes an AS event marker (indicating that the aLP sensed an intrinsic atrial event), the vLP initiates an AV interval timer. If the aLP transmits an AS event marker for all sensed events, then the vLP would preferably first determine that a PVAB or PVARP interval is not active before initiating an AV interval timer. If however the aLP transmits an AS event marker only when an intrinsic signal is sensed outside of a PVAB or PVARP interval, then the vLP could initiate the AV interval timer upon receiving an AS event marker without first checking the PVAB or PVARP status. When the aLP transmits an AP event marker (indicating that the aLP delivered or is about to deliver a pace pulse to the atrium), the vLP initiates a PVAB timer and an AV interval time, provided that a PVARP interval is not active. The vLP may also blank its sense amplifiers to prevent possible crosstalk sensing of the remote pace pulse delivered by the aLP.

**Table 4**

| "V2A" Markers / Commands (*i.e.*, from vLP to aLP) | | |
|---|---|---|
| *Marker* | *Description* | *Result in aLP* |
| VS | Notification of a sensed event in ventricle | • Initiate PVARP |
| VP | Notification of a paced event in ventricle | • Initiate PVAB |
| | | • Initiate PVARP |
| AP | Command to deliver immediate pace pulse in atrium | • Deliver immediate pace pulse to atrium |

As shown in Table 4, when the vLP senses a ventricular event, the vLP transmits an event message including a VS event marker, in response to which the aLP may initiate a PVARP interval timer. When the vLP delivers or is about to deliver a pace pulse in the ventricle, the vLP transmits VP event marker. When the aLP receives the VP event marker, the aLP initiates the PVAB interval timer and also the PVARP interval timer. The aLP may also blank its sense amplifiers to prevent possible crosstalk sensing of the remote pace pulse delivered by the vLP. The vLP may also transmit an event message containing an AP command marker to command the aLP to deliver an immediate pacing pulse in the atrium upon receipt of the command without delay.

The foregoing event markers are examples of a subset of markers that may be used to enable the aLP and vLP to maintain full dual chamber functionality. In one embodiment, the vLP may perform all dual-chamber algorithms, while the aLP may perform atrial-based hardware-related functions, such as PVAB, implemented locally within the aLP. In this embodiment, the aLP is effectively treated as a remote 'wireless' atrial pace/sense electrode. In another embodiment, the vLP may perform most but not all dual-chamber algorithms, while the aLP may perform a subset of diagnostic and therapeutic algorithms. In an alternative embodiment, vLP and aLP may equally perform diagnostic and therapeutic algorithms. In certain embodiments, decision responsibilities may be partitioned separately to one of the aLP or vLP. In other embodiments, decision responsibilities may involve joint inputs and responsibilities.

In an embodiment, ventricular-based pace and sense functionalities are not dependent on any i2i communication, in order to provide safer therapy. For example, in the event that LP to LP (i2i) communication is lost (prolonged or transient), the system 100 may automatically revert to safe ventricular-based pace/sense functionalities as the vLP device is running all of the necessary algorithms to independently achieve these functionalities. For example, the vLP may revert to a VVI mode as the vLP does not depend on i2i communication to perform ventricular pace/sense activities. Once i2i communication is restored, the system 100 can automatically resume dual-chamber functionalities.

In certain embodiments, the LP (or other type of IMD) that receives any i2i communication from another LP (or another type of IMD) or from an external system may transmit a receive acknowledgement (ACK) indicating that the receiving LP/IMD received the i2i communication, etc. In certain embodiments, a first LP (or other type of IMD) that transmits an i2i communication to a second LP (or other type of IMD) can determine that the i2i communication failed if the first LP (or other type of IMD) does not receive an ACK from the second LP (or other type of IMD) to which the i2i communication was sent within a specified window following the transmission of the i2i communication, or in a next i2i communication received from the second LP (or other type of IMD).

As explained herein, communication and synchronization between an aLP and vLP is implemented via conducted communication of event markers (per an i2i communication protocol). In certain embodiments, the i2i communication markers may be emitted only substantially concurrent with a local pace or sense event. As such, there is no risk of emitting a marker during a vulnerable period, and thus no risk of inducing unintended excitations. The i2i communication event markers are optionally expanded with a code to indicate whether the transmitting device successfully received a valid i2i marker from the remote LP since the last transmission from the remote LP. For example, a simple example of this coding uses a binary indicator (e.g., 0/1, ACK/nACK, etc.). Optionally, more sophisticated coding schemes could alternatively be employed to include expanded information, e.g., number of consecutive missed markers, handshaking to provide insight into which marker(s) were missed, etc. These "acknowledgement codes" may be used by both LPs to diagnosis bidirectional and/or unidirectional breakdowns in i2i communication, so that the LP can take remedial actions as appropriate. One such remedial action (e.g., for transient losses of i2i communication) would be to "bridge" one or more missed/corrupted markers for n future cycles before reverting to a "safeguard" mode. Another such remedial action (e.g., for more prolonged losses of i2i communication) would be to revert to a safeguard pacing mode so as to reduce and preferably minimize possibilities of asynchronous pacing by aLP and vLP.

One example of a safeguard mode is the transition from dual-chamber DDD operation functionality to ventricular-only VVI pacing functionality. The LPs would exit from safeguard mode and return to the programmed dual-chamber mode once bidirectional i2i communication has been reestablished. As another example of an alternative safeguard mode, when the LPs experience unidirectional loss of v2a i2i communication (i.e., a2v i2i communication remains intact), the aLP and vLP may transition from collectively providing DDD operation to providing VDD operation.

As noted above, when using a pair of LPs (e.g., 102, 104) to perform pacing and/or sensing operations in the RA and RV, one of the challenges is that i2i communication is relied upon to maintain appropriate synchrony between the RV and the RA. As also noted above, a transmitter (e.g., 118) of an LP 102, 104 may be configured to transmit event messages in a manner that does not inadvertently capture the heart in the chamber where LP 102, 104 is located, such as when the associated chamber is in a refractory state. In addition, an LP 102, 104 that receives an event message may enter an "event refractory" state (or event blanking state) following receipt of the event message. The event refractory/blanking state may be set to extend for a determined period of time after receipt of an event message in order to avoid the receiving LP 102, 104 from inadvertently sensing another signal as an event message that might otherwise cause retriggering. For example, the receiving LP 102, 104 may detect a conducted communication pulse from another LP 102, 104. The amplitude of a detected (i.e., sensed) conducted communication pulse can be referred to as the sensed amplitude.

As noted above, i2i conducted communication can be adversely affected by the orientation of the LPs relative to one another and the distance between the LPs. Both computer simulations and animal testing have shown that sensed amplitude varies widely with different orientation angles between LPs. For example, where a first LP (e.g., 102) transmits a pulse having a pulse amplitude of 2.5V to a second LP (e.g., 104), the sensed amplitude of the pulse received by the second LP (e.g., 104) could vary from about 2 mV to less than 0.5 mV, depending upon the orientation between the first and second LPs (e.g., 102 and 104). For example, where the LP 102 is implanted in the right atrium (RA), and the LP 104 is implanted in the left atrium (LA), e.g., as shown in FIG. 1A, the distance between and the orientation of the LPs 102 and 104 relative to one another can change over the course of each cardiac cycle. Additionally, the orientation and distance of the LPs 102 and 104 relative to one another can be affected by the posture of the patient. Accordingly, since the sensed amplitude of a pulse received by one LP (e.g., 104) from the other LP (e.g., 102) can significantly vary based on the distance between and the orientation of the LPs relative to one another, the sense amplitude can significantly vary depending upon the timing of when a pulse (a conducted communication pulse) is transmitted during a cardiac cycle, as well as the posture of the patient when the pulse is transmitted.

Assume, for example, that an LP 102, 104 has a 0.5 mV sense threshold, meaning that a sensed pulse must have an amplitude of at least 0.5 mV in order to be detected as a communication pulse by the receiving LP. In other words, if sensed amplitudes of received communication pulses are below the sense threshold, the receiving LP will fail to receive the information encoded therein and may fail to respond accordingly, which is undesirable.

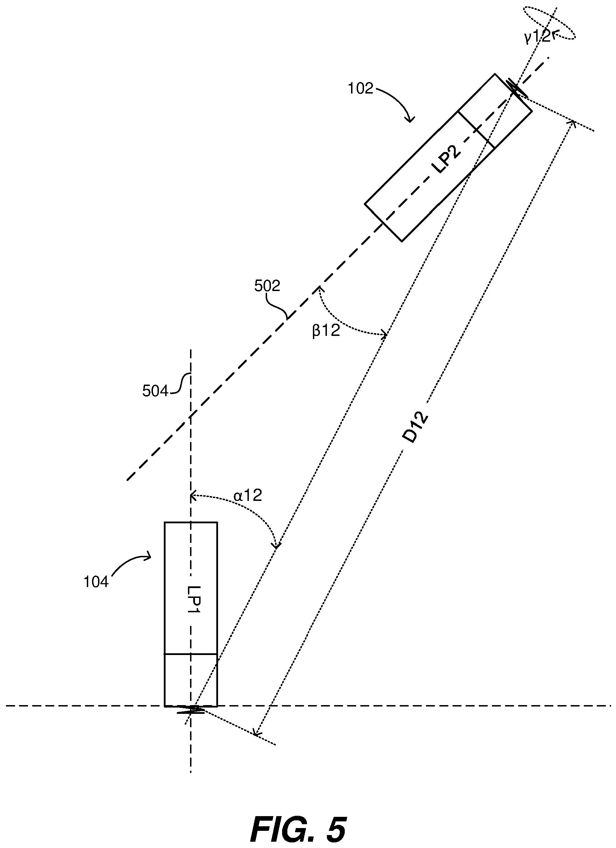

FIG. 5 is a diagram that is used to show how the orientation of two different LPs (e.g., 102, 104), labeled LP2 and LP1 in FIG. 5, can be quantified. Referring to FIG. 5, the LP2 (e.g., 102) is shown as having an axis 502, and the LP1 (e.g., 104) is shown as having an axis 504. The line D12 represents the distance between the tip electrodes of the LP1 and the LP2. In FIG. 5, the angle α12 is the angle between the axis 504 of the LP1 and the line D12; the angle β12 is the angle between the axis 502 of the LP2 and the line D12; and the angle γ12 is angle between the plane defined by the angle α12 and the plane defined by the angle β12.

Table 5, below, provides the results of simulations that show how sensed amplitudes are affected by the orientation of LP1 and LP2 relative to one another, where the LP2 is assumed to be implanted in the RA, the LP1 is assumed to be implanted in the RV, and the distance D12 is assumed to be fixed at 124 millimeters (mm). In Table 5, it is assumed that the distance D12 between the LP1 and the LP2 remains constant, so that just the orientation of LP1 and LP2 relative to one another can be analyzed. Since the distance D12 is the distance between the button (aka tip) electrodes of the LP1 and the LP2, the distance D12 can also be referred to herein as the LP1button-LP2button distance.

**Table 5**

| **Distance D12** | **Angle α12** | **Angle β12** | **RA → RV** | **RA ← RV** |
|---|---|---|---|---|
| 124 mm | 20° | 12° | 2.5 V → 2.13 mV | 2.11 mV ←2.5 V |
| 124 mm | 20° | 32° | 2.5 V → 1.82 mV | N/A |
| 124 mm | 20° | 52° | 2.5 V → 1.32 mV | N/A |
| 124 mm | 20° | 72° | 2.5 V → 0.745 mV | N/A |
| 124 mm | 20° | 82° | 2.5 V → 0.470 mV | 0.460 mV ←2.5 V |
| 124 mm | 20° | 92° | 2.5 V → 0.198 mV | 0.198 mV ←2.5 V |
| 124 mm | 10° | 82° | 2.5 V → 0.6627 mV | N/A |
| 124 mm | 40° | 82° | 2.5 V → -0.1135 mV | N/A |
| 124 mm | 50° | 82° | N/A | N/A |

The first row of Table 5 shows that when the angle β12 (i.e., the angle between the axis 502 of the LP2 and the line D12) is 12 degrees, in response to the LP2 transmitting a communication pulse having an amplitude of 2.5V, the sense amplitude of the communication pulse received by the LP1 will be 2.13 mV, which is well above a 0.5 mV sense threshold. By contrast, the sixth row of Table 5 shows that when the angle β12 is 92 degrees, in response to the LP2 transmitting a communication pulse having an amplitude of 2.5V, the sense amplitude of the communication pulse received by the LP1 will be only 0.198 mV, which is well below the 0.5 mV sense threshold. Looking at the right most column and the first row of Table 5 shows that when the angle β12 is 12 degrees, in response to the LP1 transmitting a communication pulse having an amplitude of 2.5V, the sense amplitude of the communication pulse received by the LP2 will be 2.11 mV, which is well above a 0.5 mV sense threshold; and when the angle β12 is 92 degrees, in response to the LP1 transmitting a communication pulse having an amplitude of 2.5V, the sense amplitude of the communication pulse received by the LP2 will be only 0.198 mV, which is well below the 0.5 mV sense threshold.

With larger heart sizes, the sensed amplitudes decrease. More specifically, a larger heart can cause the distance D12 between the LP1 and the LP2 to increase, with the results summarized in Table 6, below.

**Table 6**

| **Distance D12** | **Angle α12** | **Angle β12** | **RA → RV** | **RA ← RV** |
|---|---|---|---|---|
| 150 mm | 20° | 12° | 2.5 V → 0.96 mV | N/A |
| 150 mm | 20° | 32° | 2.5 V → 0.76 mV | N/A |
| 150 mm | 20° | 52° | 2.5 V → 0.51 mV | N/A |
| 150 mm | 20° | 72° | 2.5 V → 0.25 mV | N/A |
| 150 mm | 20° | 82° | 2.5 V → 0.12 mV | N/A |
| 150 mm | 20° | 92° | 2.5 V → 0.005 mV | N/A |
| 150 mm | 20° | 52° | 2.5 V → 0.51 mV | N/A |
| 150 mm | 10° | 52° | 2.5 V → 0.59 mV | N/A |
| 150 mm | 40° | 52° | 2.5V → 0.27 mV | N/A |

The results summarized in Table 6 mimic a worst case where the heart size is at the upper bounds (D12 ~ 150 mm). As can be appreciated from a comparison between Table 6 and Table 5, the sensed amplitudes decreased as D12 was increased from 124 mm to 150 mm, so that in Table 6 when the angle β12 is greater than 52 degree, the sensed amplitude is lower than the 0.5 mV sense threshold. Accordingly, it can be appreciated that conducted i2i communication between LPs implanted in larger hearts are even more adversely affected than smaller hearts by the relative orientation of the LPs. It can be appreciated from Table 6 that that i2i communication can be adversely affected by the distance between LPs.

When performing i2i communication, the one or more pulses that are transmitted from one LP to another LP can be referred more generally as the i2i signal. Due to the nature of electrode potential distribution, bipolar sensing of the i2i signal (by the LP that is receiving/sensing the i2i signal) is minimal along iso-potential lines and maximum along lines orthogonal to the iso-potential lines. In other words, when the respective axes (e.g., 502 and 504 in FIG. 5) of the two LPs (communicating with one another) are aligned with one another the sensed i2i signal is near its maximum, and when the respective axes (e.g., 502 and 504 in FIG. 5) of the two LPs are orthogonal to one another the sensed i2i signal is near its minimum.

For the purpose of this discussion, when LPs are oriented relative to another such that (for a give transmitted communication pulse amplitude) the sense amplitude of the communication pulse received by an LP will be below the sense threshold (e.g., 0.5 mV), the LPs can be said to be within a "deaf zone". This is because under such circumstances the LPs cannot successfully communicate or "hear" one another even though they are attempting to communicate or "talk" with one another.

For the purpose of this discussion, it is assumed that a dual chamber LP system includes a vLP implanted in or on a ventricular chamber (e.g., the right ventricular chamber) and an aLP implanted on or an atrial chamber (e.g., the right atrial chamber). In such an LP system, the distances between and relative orientations of the vLP and aLP varies over a cardiac cycle. When heart fills with blood, the heart is at its maximum volume and the vLP and the aLP are typically relatively far apart. By contrast, when the heart contracts, the vLP and the aLP come closer to one another and their orientation relative to one another changes. Certain embodiments of the present technology described herein leverage the fact that when a heart (in and/or on which LPs are implanted) contracts, a distance is typically reduced between the LPs, and an orientation between the LPs changes. The distance between LPs and the orientation of the LPs relative to one another are the two parameters that affect the success of conducted i2i communication.

### Monitoring and Adjusting AV Synchrony

As noted above, an aLP (e.g., 102) and a vLP (e.g., 104) can be configured to collectively provide for DDD operation when there is successful bidirectional i2i communication, i.e., when the vLP successfully receives a2v messages from the aLP, and the aLP successfully receives v2a messages from the vLP. However, for various different reasons, there may be periods of time during which bidirectional i2i communication fails, because the vLP 104 fails to successfully receive one or more a2v messages from the aLP 102, and/or the aLP 102 fails to successfully receive one or more v2a messages from the vLP 104. Such failures in i2i communication may occur, e.g., due to noise and/or the relative orientations of and/or distances between the aLP 102 and the vLP 104, but not limited thereto.

Further, as explained above in the discussion of Table 2, in a dual chamber LP system including an aLP and vLP that are configured to collectively provide DDD operation, if a2v communication is lost (i.e., one or more a2v messages transmitted by the aLP 102 are not successfully received by the vLP 104), then the vLP 104 stops tracking atrial activity, and the dual chamber LP system effectively provides DDI operation. If v2a communication is lost (i.e., one or more v2a messages transmitted by the vLP are not successfully received by the aLP), then the aLP 102 withholds pacing, and the dual chamber LP system effectively provides VDD operation. If i2i communication is simultaneously lost in both directions (i.e., v2a messages transmitted by the vLP 104 are not successfully received by the aLP 102, and a2v messages transmitted by the aLP 102 are not successfully received by the vLP 104), both mitigations take effect, and the dual chamber LP system effectively provides VDI operation. These transmission receipt safeguards act to guarantee RV pacing while maintaining RA tracking and RA pacing whenever possible.

Additionally, as noted above, certain embodiments of the present technology described herein compute an estimate of how often a dual chamber LP system (including an aLP and a vLP) provided the programmed DDD operation, as well as provided each of one or more safeguard types of operation, e.g., VDD, DDI and/or VDI. Additionally, certain embodiments of the present technology described herein compute an estimate of the time (aka cumulative duration) that the dual chamber LP system provided AV synchrony. Further, in accordance with certain embodiments of the present technology described herein, at least one parameter used by the aLP for transmitting further a2v messages to the vLP, at least one parameter used by the vLP for receiving the further a2v messages from the aLP, at least one parameter used by the vLP for transmitting further v2a messages to the aLP, and/or at least one parameter used by the aLP for receiving the further v2a messages from the vLP is adjusted based on the estimated AV synchrony. Additional details of such embodiments are now described below.

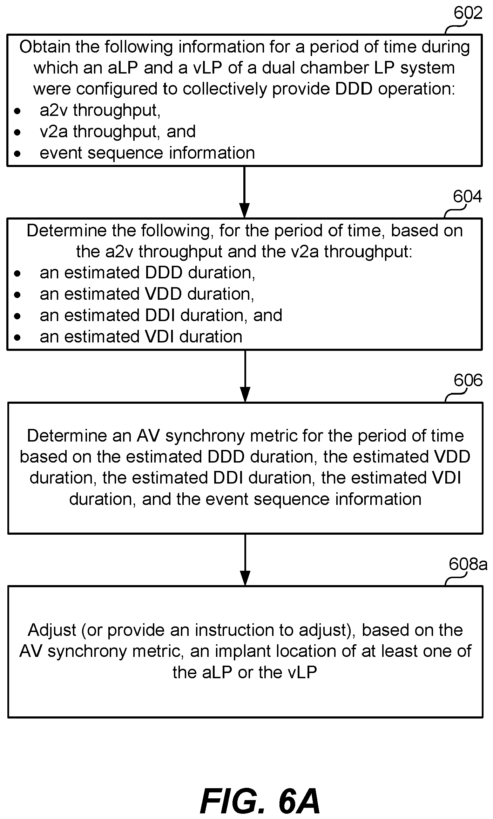

FIGS. 6A-6C are high level flow diagrams that are used to summarize certain embodiments of the present technology are used to determine and use an AV synchrony metric that specifies an estimate of how often AV synchrony was achieved during a period of time during which an aLP (e.g., 102) and a vLP (e.g., 104) of a dual chamber LP system (e.g., 100) that are configured to collectively provide DDD operation when an a2v message transmitted by the aLP is successfully received by the vLP, and a v2a message transmitted by the vLP is successfully received by the aLP, during a cardiac cycle within the period of time. Additionally, in the dual chamber LP system, the aLP and the vLP are configured to collectively provide at least one of VDD operation, DDI operation or VDI operation at least some times when at least one of an a2v message transmitted by the aLP is not successfully received by the vLP, and/or a v2a message transmitted by the vLP is not successfully received by the aLP, during a further cardiac cycle within the period of time.

As noted above, for the purpose of this discussion, the aLP is considered to have successfully received a v2a message when the aLP successfully detects and decodes the v2a message, and the vLP is considered to have successfully received an a2v message when the vLP successfully detects and decodes the v2a message.

Referring to FIG. 6A, step 602 involves obtaining a2v throughput, v2a throughput, and event sequence information, for the period of time during which the aLP and the vLP were configured to collectively provide DDD operation. The period of time can be relatively short, e.g., a few minutes, if the AV synchrony metric (determined at step 606) is used to adjust an implant location of the aLP and/or the vLP during an implant procedure. Alternatively, the period of time can be much longer, e.g., one or more hours, days, weeks, or months, but not limited thereto, if the AV synchrony metric (determined at step 606) is used to adjust one or more transmission and/or reception parameters. In accordance with certain embodiments, the period of time starts at a time when data was last cleared from memories of the aLP and the vLP, and ends when data is retrieved from the memories of the aLP and vLP. Other variations are also possible and within the scope of the embodiments described herein.

In accordance with certain embodiments, the aLP keeps track of how many a2v messages the aLP has sent to the vLP during the period of time (during which the aLP and the vLP were configured to collectively provide DDD operation), and the aLP keeps track of how many v2a message the aLP has received from the vLP during the period of time. Additionally, the vLP keeps track of how many v2a messages that vLP has sent to the aLP during the period of time, and the vLP keeps track of how many a2v message the vLP has received from the aLP during the period of time. The aforementioned information tracked by the aLP and the aforementioned information tracked by the vLP can all be uploaded to an external programmer, and based on such uploaded information, the external programmer can determine the a2v throughput, and the v2a throughput. For example, if during the period of time aLP sent one hundred a2v messages, and the vLP received ninety a2v messages, then the programmer can determine that the a2v throughput was 90%. For another example, if during the period of time the vLP sent one hundred v2a messages, and the aLP received eighty-five v2a messages, then the programmer can determine that the v2a throughput was 85%. Instead of the programmer determining the a2v throughput and the v2a throughput, it is possible that the aLP sends the information it tracks to the vLP, and the vLP uses the information it tracks plus information the aLP tracks (which was provided by the aLP to the vLP), and the vLP determines the a2v throughput and the v2a throughput. Alternatively, or additionally, it is possible that the vLP sends the information it tracks to the aLP, and the aLP uses the information it tracks plus information the vLP tracks (which was provided by the vLP to the aLP), and the aLP determines the a2v throughput and the v2a throughput. In certain embodiments, the aLP continually monitors and stores (in its memory or registers) the a2v throughput, and the vLP continually monitors and stores (in its memory or registers) the v2a throughput. In certain embodiments, the obtaining a2v throughput and the v2a throughput, at step 602, can be provided by an external system, such as an external programmer (e.g., 109) that uploads the a2v throughput from the aLP and uploads the v2a throughput from the vLP. Other types of external systems (aka non-implanted systems) that can perform step 602 include a bedside monitor, a patient link module, and a remote patient care network (PCN) that may receive such information from an external programmer, but are not limited thereto. Other variations are also possible and within the scope of the embodiments described herein, as will be appreciated from the description herein. It is noted that step 602 can include multiple sub-steps, e.g., including a sub-step that obtains a2v throughput, a sub-step that obtains v2a throughput, and a sub-step that obtains event sequence information, wherein such sub-steps need not be performed at the same time.

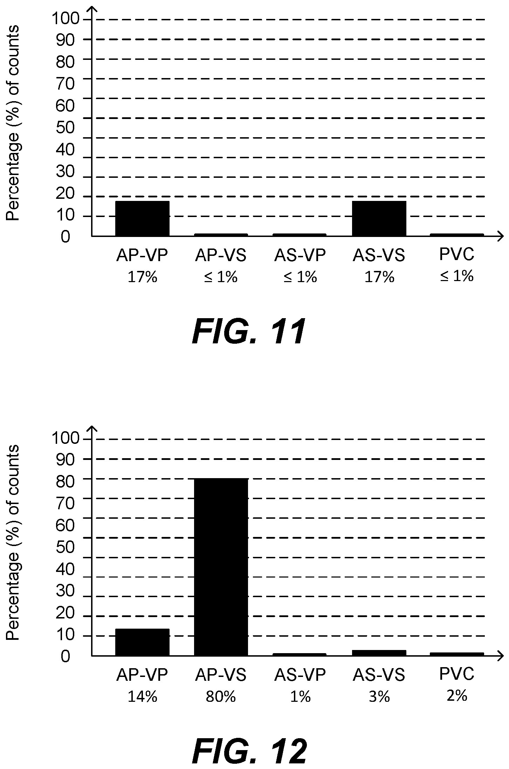

In certain embodiments, the event sequence information includes AP-VP information, AP-VS information, AS-VP information, and AS-VS information. In such embodiments, the AP-VP information specifies how often the aLP provided atrial pacing (AP) and the vLP provided ventricular pacing (VP) for a same cardiac cycle within the period of time. The AP-VS information specifies how often the aLP provided AP and the vLP provided ventricular sensing (VS) for a same cardiac cycle within the period of time. The AS-VP information specifies how often the aLP provided atrial sensing (AS) and the vLP provided VP for a same cardiac cycle within the period of time. The AS-VS information specifies how often the aLP provided AS and the vLP provided VS for a same cardiac cycle within the period of time. FIGS. 11 and 12, discussed below, are graphical representations example distributions of the event sequence information. As can be appreciated from FIGS. 11 and 12, the event sequence information may also specify how often a PVC occurred during the period of time.

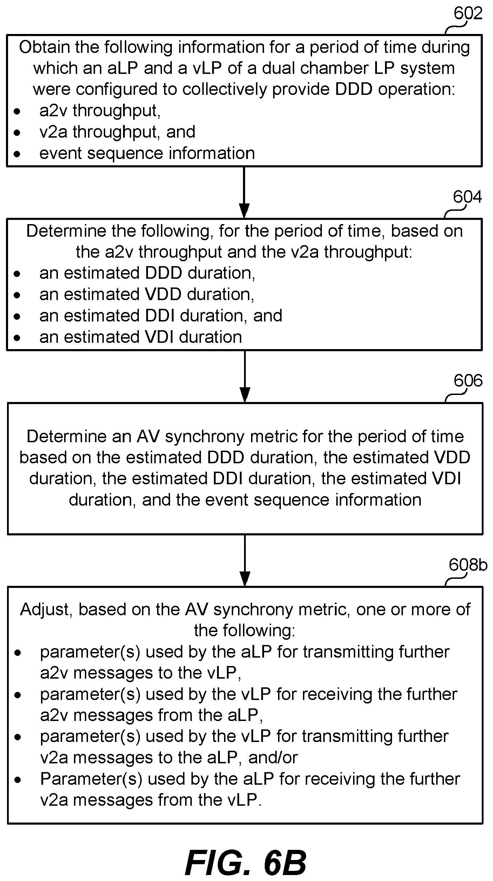

In certain embodiments, one or both of the aLP or the vLP keeps track of and stores the event sequence information. An external programmer (e.g., 109), and/or some other external system, can obtain the event sequence information from the vLP and/or the aLP when the external system interrogates or otherwise uploads information from the vLP and/or the aLP. In a specific embodiment, when the vLP and the aLP are interrogated by the external programmer the vLP transmits the event sequence information in terms of counts (AP-VP counts, AP-VS counts, AS-VP counts, and AS-VS counts) binned by atrioventricular (AV) delays (which can be used to construct an AV delay histogram) to the external programmer, and the external programmer sums up the counts for all the bins for each event sequence (AP-VP, AP-VS, AS-VP, and AS-VS) and then converts the sums of counts for each event sequence into percentages, examples of which can be seen in FIGS. 11 and 12. Other variations are also possible and within the scope of the embodiments described herein. For example, if the method summarized with reference to FIG. 6B is performed by the vLP, then there may be no need for the vLP to upload the event sequence information to an external programmer or other external system, since it would be the vLP that uses such information itself.

Still referring to FIG. 6A, step 604 involves determining, based on the a2v throughput and the v2a throughput, an estimated DDD metric, an estimated VDD metric, an estimated DDI metric, and an estimated VDI metric, for the period of time (during which the aLP and the vLP were configured to collectively provide DDD operation). In certain embodiments, the estimated DDD metric specifies an estimate of how often the aLP and the vLP collectively provided the DDD operation during the period of time during which the aLP and the vLP were configured to collectively provide the DDD operation. The estimated VDD metric specifies an estimate of how often the aLP and the vLP collectively provided the VDD operation during the period of time. The estimated DDI metric specifies an estimate of how often the aLP and the vLP collectively provided the DDI operation during the period of time. The estimated VDI metric specifies an estimate of how often the aLP and the vLP collectively provided the VDI operation during the period of time. It is noted that step 604 can include multiple sub-steps, e.g., including a sub-step that determines the estimated DDD metric, a sub-step that determines the estimated VDD metric, a sub-step that determines the estimated DDI metric, and a sub-step that determines the estimated VDI metric, wherein such sub-steps need not be performed at the same time, but rather may be performed serially one after the other, but not limited thereto.

In accordance with certain embodiments, Table 7 shown below, or the information included therein, is used to determine the estimated DDD metric, the estimated VDD metric, the estimated DDI metric, and the estimated VDI metric. More specifically, Table 7 indicates which combinations of event sequences (AP-VP, AP-VS, AS-VP, and AS-VS) and operation modes (DDD, VDD, DDI, VDI) can successfully provide AV synchrony (as represented by an "S" for Synchrony), during which the atrial and ventricular chambers of a patient's heart contract in a coordinated manner relative to one another, and which combinations of event sequences and operation modes result in AV dyssynchrony (as represented by a "D" for Dyssynchrony) during which the atrial and ventricular chambers of the patient's heart contract in an uncoordinated manner relative to one another.

**Table 7**

| **Event Sequence** | **DDD** | **VDD** | **DDI** | **VDI** |
|---|---|---|---|---|
| **AP-VP** | S | D (No AP) | S (programmed AVD) | D (non tracking mode) |
| **AP-VS** | S | D (No AP) | S | D (non tracking mode) |
| **AS-VP** | S | S | D (non tracking mode) | D (non tracking mode) |
| **AS-VS** | S | S | S | S |

As can be appreciated from Table 7, so long as the aLP and the vLP collectively provided DDD operation, then AV synchrony was achieved, regardless of which one of the AP-VP, AP-VS, AS-VP, or AS-VS event sequences was being provided. By contrast, when the aLP and the vLP collectively provided VDD operation, then AV synchrony was achieved if the AS-VP or AS-VS event sequence was provided, but not if the AP-VP or AP-VS event sequence was provided. When the aLP and the vLP collectively provided DDI operation, then AV synchrony was achieved if the AP-VP, AP-VS or AS-VS event sequence was provided, but not if the AS-VP event sequence was provided. When the aLP and the vLP collectively provide VDI operation, then AV synchrony was achieved if the AS-VS event sequence was provided, but not if the AP-VP, AP-VS, or AS-VP event sequence was provided.

Table 2, discussed above, was used to explain that in a dual chamber LP system including an aLP and a vLP that are configured to collectively provide DDD operation, if a2v communication is lost (i.e., one or more a2v messages transmitted by the aLP are not successfully received by the vLP), then the vLP stops tracking atrial activity, and the dual chamber LP system effectively provides DDI operation. If v2a communication is lost (i.e., one or more v2a messages transmitted by the vLP are not successfully received by the aLP), then the aLP withholds pacing, and the dual chamber LP system effectively provides VDD operation. If i2i communication is simultaneously lost in both directions (i.e., one or more v2a messages transmitted by the vLP are not successfully received by the aLP, and one or more a2v messages transmitted by the aLP are not successfully received by the vLP), both mitigations take effect, and the dual chamber LP system effectively provides VDI operation. These transmission receipt safeguards act to guarantee RV pacing while maintaining RA tracking and RA pacing whenever possible.

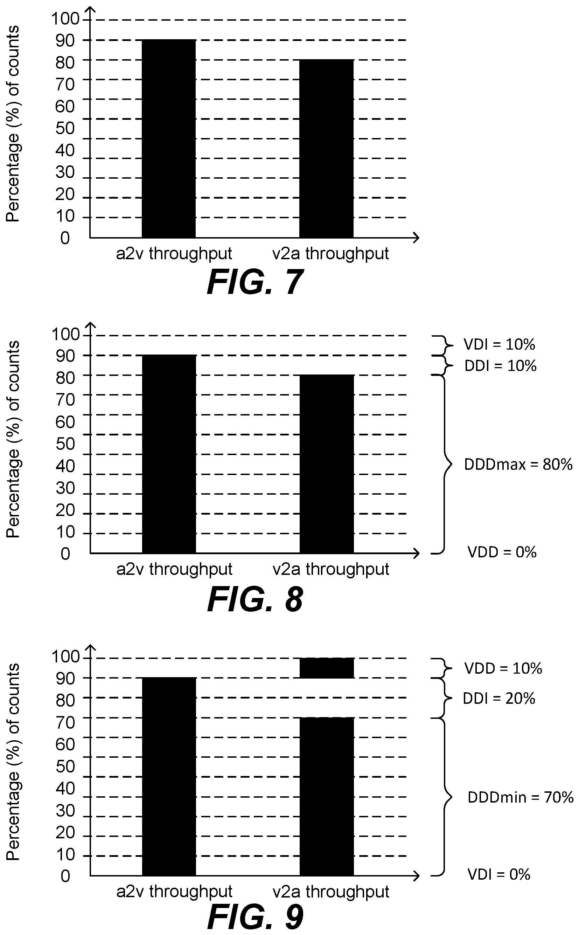

Referring briefly to FIGS. 7-9, the estimated DDD metric can correspond to an estimated DDDmax metric, an estimated DDDmin metric, and/or an estimated DDDrange. The estimated DDDmax metric, which can also be referred to herein more succinctly as DDDmax, is an estimate of the maximum of how often the dual chamber LP system achieved DDD operation during the period of time being analyzed, which estimate is based on the a2v throughput and the v2a throughput. This estimated DDDmax metric can correspond to the maximal possible overlap in successful a2v and v2a communication, which occurs when there is the minimum possible overlap in unsuccessful (i.e., loss of) a2v and v2a communication, as can be appreciated from FIG. 8. More specifically, the DDDmax metric equals the minimum of a2v throughput and v2a throughput, which can be expressed using the equation DDDmax metric = min (a2v throughput, v2a throughput). Once the DDDmax metric is determined, then a corresponding VDD metric, corresponding DDI metric, and corresponding VDI metric, can be determined. The corresponding VDD metric equals a2v throughput minus DDDmax, which can be expressed using the equation VDD metric = a2v throughput - DDDmax metric. The corresponding DDI metric equals v2a throughput minus the DDDmax metric, which can be expressed using the equation DDI metric = v2a throughput - DDDmax. The corresponding VDI metric corresponds to the remaining portion of the period during which none of DDD, VDD or DDI operation is provided. Stated another way, the corresponding VDI metric equals 100% minus the sum of the DDDmax metric, the VDD metric and the DDI metric, which can be expressed using the equation VDI metric = 100% - sum(DDDmax, VDD metric, DDI metric).

Assume, for example, that the a2v throughput equals 90% (meaning 90% of the a2v messages transmitted by the aLP are successfully received by the vLP), and the v2a throughput equals 80% (meaning 80% of the v2a messages transmitted by the vLP are successfully received by the aLP). Using the above equations, the DDDmax, VDD, DDI, and VDI metrics can be determined, as follows. DDDmax metric = min (a2v throughput, v2a throughput) = min(80%, 90%) = 80%. VDD metric = a2v throughput - DDDmax metric = 80% - 80% = 0%. DDI metric = v2a throughput - DDD max = 90% - 80% = 10%. VDI metric = 100% - sum(DDDmax, VDD metric, DDI metric) = 100% - sum(80% + 0% + 10%) = 100% - 90% = 10%.

As noted above, the estimated DDD metric can instead (or additionally) correspond to an estimated DDDmin metric, which can also be referred to herein more succinctly as DDDmin, and is an estimate of the minimum of how often the dual chamber LP system achieved DDD operation during the period of time being analyzed, which estimate is based on the a2v throughput and the v2a throughput. This estimated DDDmin metric can correspond to the minimum possible overlap in successful a2v and v2a communication, which occurs when there is the maximum possible overlap in unsuccessful (i.e., loss of) a2v and v2a communication, as can be appreciated from FIG. 9. More specifically, the DDDmin metric equals the maximum of zero and 100% minus a sum of %a2v messages that were not successfully received by the vLP and %v2a messages that were not successfully received by the aLP, which can be expressed using the equation DDDmin metric = max (0, 100% - [(100% - a2v throughput) + (100% - v2a throughput)]. The corresponding VDD metric is equal to the a2v throughput minus the estimated DDDmin metric, which can be expressed using the equation VDD metric = a2v throughput - DDDmin. The corresponding DDI metric is equal to the v2a throughput minus the estimated DDDmin metric, which can be expressed using the equation DDI metric = v2a throughput - DDDmin. The corresponding VDI metric corresponds to the remaining portion of the period during which none of DDD, VDD or DDI operation is provided. Stated another way, the corresponding VDI metric is equal to 100% minus a sum of the DDDmin metric, the VDD metric, and the DDI metric, which can be expressed using the equation VDI metric = 100% - sum(DDDmin, VDD metric, DDI metric).

Assume again for example, that the a2v throughput equals 90% (meaning 90% of the a2v messages transmitted by the aLP are successfully received by the vLP), and the v2a throughput equals 80% (meaning 80% of the v2a messages transmitted by the vLP are successfully received by the aLP). Using the above equations, the DDDmin, VDD, DDI, and VDI metrics can be determined, as follows. DDDmin metric = max (0, 100% - [(100% - a2v throughput) + (100% - v2a throughput)] = max (0, 100% - [100%-90% + 100-80%]) = max (0, 100% - [10% + 20%]) = max (0, 70%) = 70%. VDD metric = a2v throughput - DDDmin metric = 80% - 70% = 10%. DDI metric = v2a throughput - DDDmin = 90% - 70% = 20%. VDI metric = 100% - sum(DDDmin, VDD metric, DDI metric) = 100% - sum(70% + 10% + 20%) = 100% - 100% = 0%.

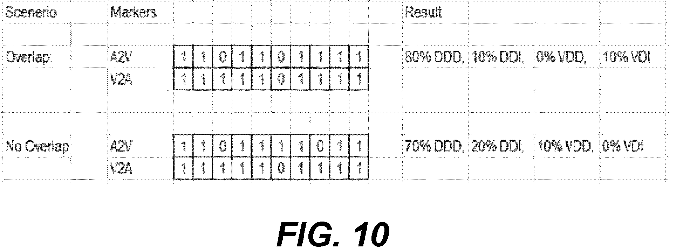

The schematic shown in FIG. 10 helps visualize the temporal overlap options for successful a2v communication and successful v2a communication. The schematic uses the a2v and v2a rows with 10 squares collectively representing a total of 100% of the duration, and each of the 10 squares representing 10% of the duration. In each square, a value of 1 indicates successful i2i communication and a value 0 indicates unsuccessful i2i communication.

Referring again to FIG. 6A, step 606 involves determining an AV synchrony metric for the period of time (during which the aLP and the vLP were configured to collectively provide DDD operation) based on the estimated DDD metric, the estimated VDD metric, the estimated DDI metric, the estimated VDI metric, and the event sequence information. The estimated DDD metric used at step 606 can be the estimated DDDmax metric, in which case, the corresponding estimates of the VDD, DDI, and DDI metrics are used. Alternatively, estimated DDD metric used at step 606 can be the estimated DDDmin metric, in which case, the corresponding estimates of the VDD, DDI, and DDI metrics are used. It would also be possible to determine both the DDDmax metric (and its corresponding VDD, DDI, and DDI metrics) and the DDDmin metric (and its corresponding VDD, DDI, and DDI metrics), and use each of these metrics at step 606 to determine a respective AV synchrony metric, one of which specifies a maximum possible AV synchrony and the other of which specifies a minimum possible AV synchrony, and which can collectively specify an AV synchrony range. The AV synchrony metric, as noted above, specifies an estimate of how often AV synchrony was achieved during the period of time.

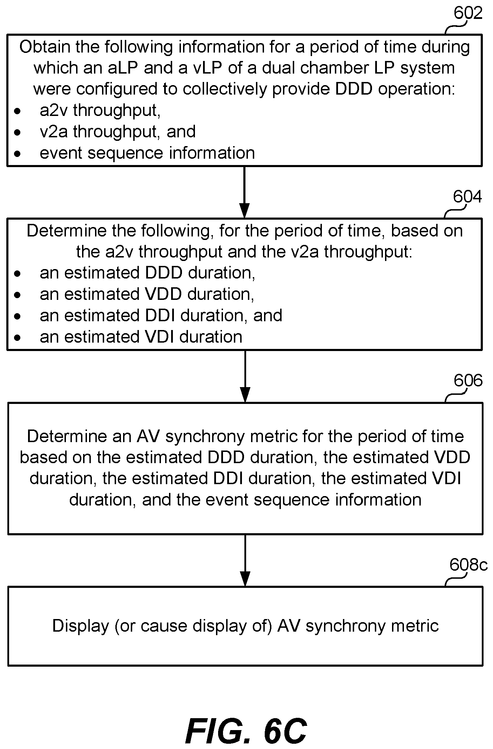

In accordance with certain embodiments, the event sequence information (obtained at step 602, and used at step 606) includes AP-VP information, AP-VS information, AS-VP information, and AS-VS information. The AP-VP information specifies how often the aLP provided atrial pacing (AP) and the vLP provided ventricular pacing (VP) for a same cardiac cycle within the period of time. The AP-VS information specifies how often the aLP provided AP and the vLP provided ventricular sensing (VS) for a same cardiac cycle within the period of time. The AS-VP information specifies how often the aLP provided atrial sensing (AS) and the vLP provided VP for a same cardiac cycle within the period of time. The AS-VS information specifies how often the aLP provided AS and the vLP provided VS for a same cardiac cycle within the period of time. Additionally details of step 606, according to certain embodiments of the present technology, are provided below following the discussion of step 608a, as well as following the discussions of steps 608b and 608c of FIGS. 6B and 6C.

Referring again to FIG. 6A, step 608a involves adjusting, based on the AV synchrony metric, an implant location of the aLP and/or the vLP. For example, the method summarized with reference to FIG. 6A can be performed, e.g., by an external programmer (e.g., 109), during an implant procedure, and used to determine whether an implant location of the aLP and/or the vLP provides for at least a threshold level of AV synchrony. Assume for example that the threshold level of AV synchrony is 60%. After an aLP (e.g., 102) and a vLP (e.g., 104) are implanted using a catheter system in a patient's heart (e.g., 101), steps 602, 604 and 606 can be performed to determine an AV synchrony metric (at step 606), which can then be compared to the threshold level of AV synchrony. If the threshold level of AV synchrony is reached, then there may be a determination that there is no need to adjust the implant location of the aLP and/or the implant location of the vLP. However, if the threshold level of AV synchrony is not reached, then there may be a determination that there is a need to adjust the implant location of the aLP and/or the implant location of the vLP, after which steps 602, 604 and 606 can be repeated to determine a new AV synchrony metric (at another instance of step 606).

Alternative or additional steps, that can be provided instead of (or in addition to) step 608a, are described below with reference to FIGS. 6B and 6C. Indeed, steps 602, 604 and 606 in FIGS. 6B and 6C are identical to those commonly numbered steps in FIG. 6A, and thus, when discussing FIGS. 6B and 6C, details of steps 602, 604 and 606 (already provided above) need not be repeated. Following the below discussion of FIGS. 6B and 6C, further details of step 606, according to certain embodiments of the present technology, are provided.

Referring now to FIG. 6B, in accordance with certain embodiments, after the AV synchrony metric is determined at step 606, step 608b involves adjusting, based on the AV synchrony metric, at least one parameter used by the aLP for transmitting further a2v messages to the vLP, at least one parameter used by the vLP for receiving the further a2v messages from the aLP, at least one parameter used by the vLP for transmitting further v2a messages to the aLP, and/or at least one parameter used by the aLP for receiving the further v2a messages from the vLP. Such parameters are examples of i2i communication parameters that are capable of being programmed and adjusted, and which when adjusted may affect that resulting AV synchrony.

For example, steps 602, 604 and 606 can be performed while default or programmed i2i communication parameters are used, in order to determine whether the AV synchrony using such i2i communication parameters provide for at least a threshold level of AV synchrony. Assume again for example that the threshold level of AV synchrony is 60%. If the threshold level of AV synchrony is reached using the default or programmed i2i communication parameters, then there may be a determination that there is no need to adjust any of the i2i communication parameters. However, if the threshold level of AV synchrony is not reached, then there may be a determination that there is a need to adjust one or more i2i communication parameters, and more specifically, at least one parameter used by the aLP for transmitting further a2v messages to the vLP, at least one parameter used by the vLP for receiving the further a2v messages from the aLP, at least one parameter used by the vLP for transmitting further v2a messages to the aLP, and/or at least one parameter used by the aLP for receiving the further v2a messages from the vLP. Parameters used by the aLP for transmitting further a2v messages to the vLP, which may be adjusted (e.g., increased), include i2i communication pulse amplitude, and/or i2i communication pulse width, but are not limited thereto. Similarly, parameters used by the vLP for transmitting further v2a messages to the aLP, which may be adjusted (e.g., increased), include i2i communication pulse amplitude, and/or i2i communication pulse width, but are not limited thereto. Parameters used by the vLP for receiving further a2v messages from the aLP, which may be adjusted, include a gain of a receive amplifier, a communication pulse sense threshold, or more generally, a receive communication sensitivity parameter, but are not limited thereto. Similarly, parameters used by the aLP for receiving further v2a messages from the vLP, which may be adjusted, include a gain of a receive amplifier, a communication pulse sense threshold, or more generally, a receive communication sensitivity parameter, but not limited thereto.

As explained above with reference to FIG. 5, the sensed amplitude of an i2i communication pulse received by one LP (e.g., 104) from the other LP (e.g., 102) can significantly vary based on the orientation of the LPs relative to one another. Accordingly, a sense amplitude of an i2i communication pulse can significantly vary depending upon the timing of when i2i communication pulses are transmitted within a cardiac cycle, as well as the posture of the patient when the pulses are transmitted. In accordance with certain embodiments of the present technology, the timing of when v2a messages are transmitted within cardiac cycles from the vLP to the aLP, and/or the timing of when a2v messages are transmitted within cardiac cycles from the aLP to the vLP, can be adjusted to attempt to increase v2a throughput and/or a2v throughput for the purpose of attempting to improve AV synchrony.

Rather than, or in addition to adjusting one or more i2i communication parameters to provide for at least a threshold level of AV synchrony, various combinations of i2i communication parameters can be tested to optimize (e.g., attempt to maximize) AV synchrony. In accordance with certain embodiments, the method summarized with reference to FIG. 6B is performed to determine what combination of i2i communication parameters (examples of which were described above) provide for least a threshold level of AV synchrony while minimizing energy consumption, or more generally, combinations of i2i communication parameters can be tested to determine which combination provides for a good tradeoff between AV synchrony and power consumption. In still other embodiments, where the desire is to improve and preferably maximize device longevity, combinations of i2i communication parameters can be tested to determine which combination provides for the lowest power consumption while still providing for at least a threshold level of AV synchrony. Other variations are also possible and within the scope of the embodiments described herein.

Referring now to FIG. 6C, in accordance with certain embodiments, after the AV synchrony metric is determined at step 606, step 608c involves displaying the determined AV synchrony metric to a clinician on a display. Step 608c can be provided on its own, or together with one or more of steps 608a and 608b.

Once the AV synchrony metric is determined, a processor (or a clinician) can then make determinations based on the AV synchrony metric, such as whether one or both of the aLP and/or vLP should be replaced, repositioned or reprogrammed, or a combination thereof. For example, embodiments of the present technology described herein can be used to determine that even though i2i communication is poor in one direction, acceptable AV synchrony is still achieved for certain patient's, thereby indicating that there is no need to replace, reposition and/or reprogram the aLP and the vLP.

Alternatively, or additionally, AV synchrony metrics determined at separated instances of step 606, determined over an extended period of time, can be stored and used to track (aka monitor) disease progression and/or device malfunction in general. Tracking of disease progression based on AV synchrony metrics can be used to adjust (e.g., increase and/or change) a patient's medication, or the like. Tracking of device malfunction can be used to determine when at least one of the aLP or vLP needs to be repositioned, replaced, or reprogrammed, but not limited thereto. Other uses of the AV synchrony metrics are also possible and within the scope of the embodiments described herein.

Additional details of step 606 (in FIGS. 6A, 6B and 6C) are now described. In accordance with certain embodiments, at step 606, the event sequence distribution is used in conjunction with the percentages of the safeguard modes (DDI, VDD, and VDI) associated with DDDmin or DDDmax, and an estimated minimum % AV synchrony is determined. The technique is the same for both DDDmin and DDDmax, but the values are different, as shown previously, and the resulting estimate using DDDmin is lower than with DDDmax. The example calculations shown below used the values for DDDmin.

In accordance with certain embodiments, determining the AV synchrony metric at step 606 involves determining the maximum % AV dyssynchrony during VDI operation, which depends on the event sequence distribution (e.g., AP-VS%, AP-VP%, and AS-VP%). At the time when step 606 is performed, the estimated DDD, VDD, DDI, and VDI metrics (e.g., DDD%, VDD%, DDI%, and VDI%) were already determined at step 604 (based on the a2v throughput and v2a throughput obtained at step 602), and the event sequence information (e.g., AP-VP%, AP-VS%, AS-VP%, and AS-VS%) is already known. However, when step 606 is performed, it is not known (aka it is unknown) how the estimated DDD, VDD, DDI, and VDI metrics (e.g., DDD%, VDD%, DDI%, and VDI%) overlapped with the event sequence information (e.g., AP-VP%, AP-VS%, AS-VP%, and AS-VS%). More specifically, it is not known how often the AP-VP event sequence overlapped with each of DDD, VDD, DDI, and VDI; it is not known how often the AP-VS event sequence overlapped with each of DDD, VDD, DDI, and VDI; it is not known how often the AS-VP event sequence overlapped with each of DDD, VDD, DDI, and VDI; and it is not known how often the AS-VS event sequence overlapped with each of DDD, VDD, DDI, and VDI. Accordingly, in accordance with certain embodiments, an estimate of AV synchrony is determined for all these various possible combinations of how the estimated DDD, VDD, DDI, and VDI metrics (e.g., DDD%, VDD%, DDI%, and VDI%) overlap with the event sequence information (e.g., AP-VP%, AP-VS%, AS-VP%, and AS-VS%), and the calculated AV synchrony that resulted in the worst case scenario for AV synchrony is selected as the of AV synchrony metric determined at step 606. Example details for implementing step 606 are described below. One of ordinary skill in the art reading the below description will appreciate that step 606 can be achieved in other similar manners that are also within the scope of the embodiments described herein.

First, the distributions of all the relevant event sequences, AP-VS/AP-VP/AS-VP, are combined to get the minimum between this and the VDI%, which result is the maximum dyssynchrony while providing VDI operation. Next, while the temporal overlap between event sequences and periods of VDI operation may be unknown, the following determinations can be provided for each of six possible permutations of event sequence order, and the permutation resulting in maximum % AV dyssynchrony is selected. The six possible permutations of event sequence order are as follows: Sequence #1: AP-VS%, AP-VP%, AS-VP%; Sequence #2: AP-VS%, AS-VP%, AP-VP%, Sequence #3: AP-VP%, AS-VP%, AP-VS%; Sequence #4: AP-VP%, AP-VS%, AS-VP%, Sequence #5: AS-VP%, AP-VS%, AP-VP%; and Sequence #6: AS-VP%, AP-VP%, AP-VS%.

For a given event sequence (e.g., Sequence #1 above), the first event % (e.g., AP-VS%) is subtracted from the VDI% to obtain any remaining % in the 1st event. Vice versa, the VDI% is subtracted from the first event to obtain any remaining VDI%. With the remaining VDI% the subtraction is provided with the 2nd event, and again vice versa, to obtain any remaining % in the 2nd event and remaining in VDI%. This is again provided for the 3rd event, to thereby obtain any remaining % in the 3rd event.

The algorithm proceeds with the remaining of each event percentage. The minimum between the sum of event sequences AP-VS% and AP-VP% and the VDD% is determined, which result is the maximum AV dyssynchrony while providing VDD operation. Next, the minimum between the AS-VP% and the DDI% is determined, which result is the maximum AV dyssynchrony while providing DDI operation. Then, the AV dyssynchrony percentages while providing VDI, VDD, and DDI operation are summed and subtracted from 100% to calculate the estimated minimum AV synchrony percentage for this particular event sequence order. Finally, after performing this calculation for all 6 possible event sequence permutations, the minimum from the 6 permutations is selected as the estimated minimum AV synchrony, which is an example of the AV synchrony metric determined at step 606. This example shows how combining the event sequence information with the a2v throughput and v2a throughput can result in an approximation of AV synchrony, which can then be used at step 608a, 608b, and/or step 608c, as well as in other manners.

Below is an example of the determinations (e.g., calculations) using the following distribution of operation modes achieved by the dual chamber LP system and of event sequences: DDD(min or max) = 60%; VDD = 15%; DDI = 5%; VDI = 20%; AP-VS = 35%; AP-VP = 40%; AS-VP = 25%; and AS-VS = 0%.

For the VDI safeguard operation mode, assuming VDI = 20%, the duration in AV dyssynchrony (dys) for event sequences AP-VS/AP-VP/AS-VP = 35% + 40% + 25% = 100%. VDI_{dys} maximum AV dyssynchrony % = min (20%, 100%) = min (20, 100) = 20%. Calculations are then provided based on the order of event sequence.

For event sequence order #1: AP-VS, AP-VP, AS-VP, the duration remaining in AP-VS = max(AP-VS% - VDI_{dys} maximum AV dyssynchrony %, 0) = max(35 - 20, 0) = 15. The remaining VDI duration after overlap with AP-VS = max(VDI_{dys} maximum AV dyssynchrony % - AP-VS%, 0) = max(20 - 35, 0) = 0%. The duration remaining in AP-VP = max(AP-VP% - Remaining VDI after overlap with AP-VS, 0) = max(40 - 0, 0) = 40. The remaining VDI after overlap with AP-VP = max(remaining VDI after overlap with AP-VS - AP-VP%, 0) = max(0 - 40, 0) = 0. The duration remaining in AS-VP = max(AS-VP% - remaining VDI after overlap with AP-VP, 0) = max(25 - 0, 0) = 25.

For the VDD safeguard operation mode, assuming VDD = 15%, the duration in AV dyssynchronous event sequences AP-VS/AP-VP (remaining) = Remaining AP-VS (as determined above) + Remaining AP-VP (as determined above) = 15% + 40% = 55%. The VDD_{dys} maximum AV dyssynchrony % = min (VDD, duration in AV dyssynchronous event sequences AP-VS/AP-VP) = min (15, 55) = 15%.

For the DDI safeguard mode, assuming DDI = 5%, the duration in AV dyssynchronous event sequences AS-VP = Remaining AS-VP (as determined above) = 25%. The DDI_{dys} maximum AV dyssynchrony % = min (DDI, duration in AV dyssynchronous event sequences AS-VP) = min (5, 25) = 5%.

Continuing with the above example calculations, the total estimated minimum % AV synchrony = 100% - sum(VDI_{dys}, VDD_{dys}, DDI_{dys}) = 100% - sum(20, 15, 5) = 60%. Under these conditions (distributions of modes and event sequences), the estimated minimum AV synchrony would have been 60% for the patient. The actual AV synchrony may have been higher. This example shows how combining the Event Sequence and i2i can result in an approximation of AV synchrony. This approximation can be presented to customers to show a range of AV synchrony customized to the patient

Additional assumptions can be made about the overlap of the i2i communication loss (a2v and/or v2a communication loss) and the event sequences, with a simplification that the event sequence distribution is similar temporally. This implies that the overlap between the safeguard modes and the need for an event sequence does not have to be the worst case estimate allowing for determining a range for AV synchrony.

In certain embodiments, the aLP (e.g., 102) can be programmed to pace in the atrium, given atrial pacing demand, despite concurrent loss of a2v communication. This is called a "bridged" cycle and was not included in the calculations provided in the above examples. The bridged cycle essentially maintains DDD mode during v2a i2i communication loss, and, by default, it currently is only activated for the first cycle/beat of each v2a i2i communication loss occurrence (but it could potentially be extended to 2 or more cycles). The bridged cycle diagnostic can be considered to add to the v2a throughput diagnostic. The calculation is provided by summing the counts of v2a message receipts and the bridged cycle counts, then dividing by the v2a message transmissions. The example calculations provided above can be modified to account for bridge cycles, but instead use a v2a throughput of 75%. Then if there were bridged cycles at a 15% rate, then the effective v2a throughput would be 90% (75% + 15%). The rest of the calculations in the previous example would remain the same.

In accordance with certain embodiments, at least one of the vLP or the aLP maintains a histogram count of the number of transmitted v2a messages that were not successfully received by the aLP and/or the number of transmitted a2v messages that were not successfully received by the vLP, with the histogram binned by loss duration. The duration information is summed to get a total loss duration. For this total loss duration, the number of i2i transmit messages (TX) that are received is ~0. To allocate the number of TX for this duration, an average heart rate for the patient is determined using the data from a heart rate histogram, as the LP sends a TX for each heart beat. The calculation is split into two periods, the loss period and the regular period. The loss period is a ratio of the number of i2i transmit messages (TX) during the loss period that did not have a RX message over all TX for the LP. The regular period is 1 - loss period.

For the loss period, the distribution of operation modes achieved are calculated by assuming 0% DDD, and then calculating whether the i2i loss periods overlapped or were sequential. This results in two sets of VDD, DDI and VDI for the sequential (where VDI will be minimized) and overlap calculations (where VDI will be maximized). The total i2i loss period ratio for the two sets of sequential mode calculation and overlap mode calculation is used to create the corresponding regular period ratios. The i2i % performance for the regular period is recalculated by decreasing the number of TX that were sent during the loss period, and then computing RX/TX for each direction, wherein during the regular period the TX in the denominator has a smaller magnitude, while the RX count in the numerator stayed effectively the same. The distribution of modes is then calculated as described above, but using the new i2i performance values. The resulting distribution of modes will then be weighted by the regular period which is the equal to 100% minus the loss period ratio. For each mode in the distribution, the loss period and the regular period are summed to provide a total % spent in the mode, which is then used to assess the impact on AV Synchrony as further described above.

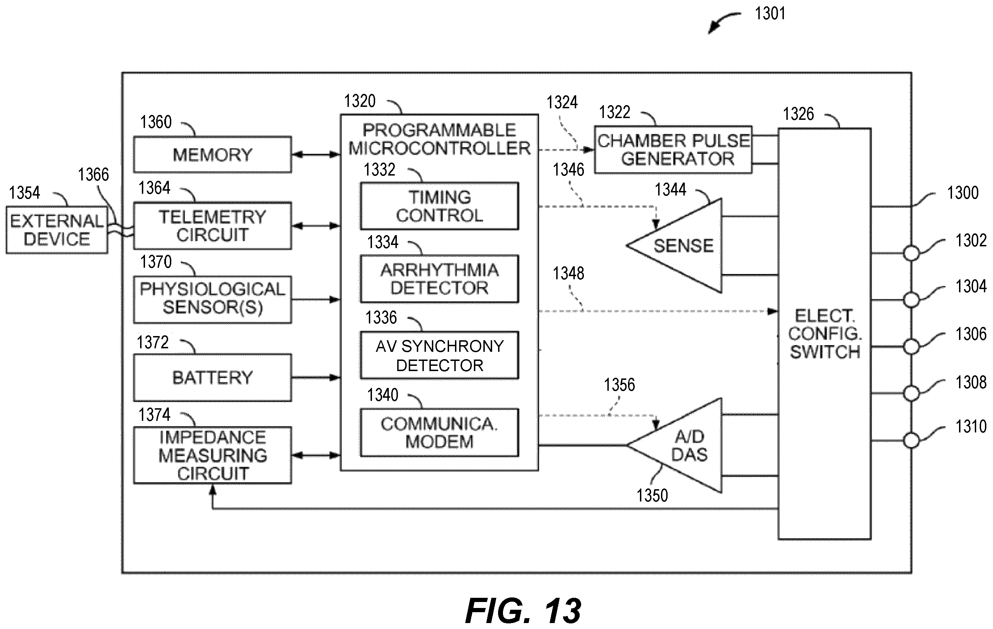

FIG. 13 shows a block diagram of one embodiment of an LP 1301 (e.g., 102 or 104) that is implanted into the patient as part of the implantable cardiac system in accordance with certain embodiments herein. LP 1301 may be implemented as a full-function biventricular pacemaker, equipped with both atrial and ventricular sensing and pacing circuitry for four chamber sensing and stimulation therapy (including both pacing and shock treatment). Optionally, LP 1301 may provide full-function cardiac resynchronization therapy. Alternatively, LP 1301 may be implemented with a reduced set of functions and components.

LP 1301 has a housing 1300 to hold the electronic/computing components. Housing 1300 (which is often referred to as the "can", "case", "encasing", or "case electrode") may be programmably selected to act as the return electrode for certain stimulus modes. Housing 1300 may further include a connector (not shown) with a plurality of terminals 1302, 1304, 1306, 1308, and 1310. The terminals may be connected to electrodes that are located in various locations on housing 1300 or elsewhere within and about the heart. LP 1301 includes a programmable microcontroller 1320 that controls various operations of LP 1301, including cardiac monitoring and stimulation therapy. Microcontroller 1320 includes a microprocessor (or equivalent control circuitry), RAM and/or ROM memory, logic and timing circuitry, state machine circuitry, and I/O circuitry.

LP 1301 further includes a pulse generator 1322 that generates stimulation pulses and communication pulses for delivery by one or more electrodes coupled thereto. Pulse generator 1322 is controlled by microcontroller 1320 via control signal 1324. Pulse generator 1322 may be coupled to the select electrode(s) via an electrode configuration switch 1326, which includes multiple switches for connecting the desired electrodes to the appropriate I/O circuits, thereby facilitating electrode programmability. Switch 1326 is controlled by a control signal 1328 from microcontroller 1320.

In the embodiment of FIG. 13, a single pulse generator 1322 is illustrated. Optionally, the IMD may include multiple pulse generators, similar to pulse generator 1322, where each pulse generator is coupled to one or more electrodes and controlled by microcontroller 1320 to deliver select stimulus pulse(s) to the corresponding one or more electrodes.

Microcontroller 1320 is illustrated as including timing control circuitry 1332 to control the timing of the stimulation pulses (e.g., pacing rate, atrio-ventricular (AV) delay, atrial interconduction (A-A) delay, or ventricular interconduction (V-V) delay, etc.). Timing control circuitry 1332 may also be used for the timing of refractory periods, blanking intervals, noise detection windows, evoked response windows, alert intervals, marker channel timing, and so on. Microcontroller 1320 also has an arrhythmia detector 1334 for detecting arrhythmia conditions. Microcontroller 1320 also has an AV synchrony detector 1336 that can be configured to determine an AV synchrony metric in accordance with embodiments of the present technology described herein. Although not shown, the microcontroller 1320 may further include other dedicated circuitry and/or firmware/software components that assist in monitoring various conditions of the patient's heart and managing pacing therapies. The microcontroller can include a processor. The microcontroller, and/or the processor thereof, can be used to perform the methods of the present technology described herein.

LP 1301 is further equipped with a communication modem (modulator/demodulator) 1340 to enable wireless communication with the remote slave pacing unit. Modem 1340 may include one or more transmitters and two or more receivers as discussed herein in connection with FIG. 1B. In one implementation, modem 1340 may use low or high frequency modulation. As one example, modem 1340 may transmit i2i messages and other signals through conducted communication between a pair of electrodes. Modem 1340 may be implemented in hardware as part of microcontroller 1320, or as software/firmware instructions programmed into and executed by microcontroller 1320. Alternatively, modem 1340 may reside separately from the microcontroller as a standalone component.

LP 1301 includes a sensing circuit 1344 selectively coupled to one or more electrodes, that perform sensing operations, through switch 1326 to detect the presence of cardiac activity in the right chambers of the heart. Sensing circuit 1344 may include dedicated sense amplifiers, multiplexed amplifiers, or shared amplifiers. It may further employ one or more low power, precision amplifiers with programmable gain and/or automatic gain control, bandpass filtering, and threshold detection circuit to selectively sense the cardiac signal of interest. The automatic gain control enables the unit to sense low amplitude signal characteristics of atrial fibrillation. Switch 1326 determines the sensing polarity of the cardiac signal by selectively closing the appropriate switches. In this way, the clinician may program the sensing polarity independent of the stimulation polarity.

The output of sensing circuit 1344 is connected to microcontroller 1320 which, in turn, triggers or inhibits the pulse generator 1322 in response to the presence or absence of cardiac activity. Sensing circuit 1344 receives a control signal 1346 from microcontroller 1320 for purposes of controlling the gain, threshold, polarization charge removal circuitry (not shown), and the timing of any blocking circuitry (not shown) coupled to the inputs of the sensing circuitry.

In the embodiment of FIG. 13, a single sensing circuit 1344 is illustrated. Optionally, the IMD may include multiple sensing circuits, similar to sensing circuit 1344, where each sensing circuit is coupled to one or more electrodes and controlled by microcontroller 1320 to sense electrical activity detected at the corresponding one or more electrodes. Sensing circuit 1344 may operate in a unipolar sensing configuration or in a bipolar sensing configuration.

LP 1301 further includes an analog-to-digital (A/D) data acquisition system (DAS) 1350 coupled to one or more electrodes via switch 1326 to sample cardiac signals across any pair of desired electrodes. Data acquisition system 1350 is configured to acquire intracardiac electrogram signals, convert the raw analog data into digital data, and store the digital data for later processing and/or telemetric transmission to an external system 1354 (e.g., a programmer, local transceiver, or a diagnostic system analyzer). Data acquisition system 1350 is controlled by a control signal 1356 from the microcontroller 1320.

Microcontroller 1320 is coupled to a memory 1360 by a suitable data/address bus. The programmable operating parameters used by microcontroller 1320 are stored in memory 1360 and used to customize the operation of LP 1301 to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each shocking pulse to be delivered to the patient's heart within each respective tier of therapy. The memory 1360 can also be used to store one or more of a2v throughput, v2a throughput, event sequence information, and/or one or more AV synchrony metrics.

The operating parameters of LP 1301 may be non-invasively programmed into memory 1360 through a telemetry circuit 1364 in telemetric communication via communication link 1366 with external system 1354. Telemetry circuit 1364 allows intracardiac electrograms and status information relating to the operation of LP 1301 (as contained in microcontroller 1320 or memory 1360) to be sent to external system 1354 through communication link 1366.

LP 1301 can further include magnet detection circuitry (not shown), coupled to microcontroller 1320, to detect when a magnet is placed over the unit. A magnet may be used by a clinician to perform various test functions of LP 1301 and/or to signal microcontroller 1320 that external system 1354 is in place to receive or transmit data to microcontroller 1320 through telemetry circuits 1364.

LP 1301 can further include one or more physiological sensors 1370. Such sensors are commonly referred to as "rate-responsive" sensors because they are typically used to adjust pacing stimulation rates according to the exercise state of the patient. However, physiological sensor 1370 may further be used to detect changes in cardiac output, changes in the physiological condition of the heart, or diurnal changes in activity (e.g., detecting sleep and wake states). Signals generated by physiological sensors 1370 are passed to microcontroller 1320 for analysis. Microcontroller 1320 responds by adjusting the various pacing parameters (such as rate, AV Delay, V-V Delay, etc.) at which the atrial and ventricular pacing pulses are administered. While shown as being included within LP 1301, physiological sensor(s) 1370 may be external to LP 1301, yet still be implanted within or carried by the patient. Examples of physiologic sensors might include sensors that, for example, sense respiration rate, pH of blood, ventricular gradient, activity, position/posture, minute ventilation (MV), and so forth.

A battery 1372 provides operating power to all of the components in LP 1301. Battery 1372 is capable of operating at low current drains for long periods of time, and is capable of providing high-current pulses (for capacitor charging) when the patient requires a shock pulse (e.g., in excess of 2 A, at voltages above 2 V, for periods of 10 seconds or more). Battery 1372 also desirably has a predictable discharge characteristic so that elective replacement time can be detected. As one example, LP 1301 employs lithium/silver vanadium oxide batteries.

LP 1301 further includes an impedance measuring circuit 1374, which can be used for many things, including: lead impedance surveillance during the acute and chronic phases for proper lead positioning or dislodgement; detecting operable electrodes and automatically switching to an operable pair if dislodgement occurs; measuring respiration or minute ventilation; measuring thoracic impedance for determining shock thresholds; detecting when the device has been implanted; measuring stroke volume; and detecting the opening of heart valves; and so forth. Impedance measuring circuit 1374 is coupled to switch 1326 so that any desired electrode may be used.

Processes for chamber identification may also be applied to subcutaneous pacemakers, ICDs, with leads and the like. A device with one or more implanted leads, identification and/or confirmation of the chamber into which the lead was implanted could be useful in several pertinent scenarios. For example, for a DR or CRT device, automatic identification and confirmation could mitigate against the possibility of the clinician inadvertently placing the V lead into the A port of the implantable medical device, and vice-versa. As another example, for an SR device, automatic identification of implanted chamber could enable the device and/or programmer to select and present the proper subset of operation modes (e.g., AAI or VVI), and for the IPG to utilize the proper set of settings and algorithms (e.g., V-AutoCapture vs ACap-Confirm, sensing sensitivities, etc.).

FIG. 14 illustrates an example system that can be used to determine, display and otherwise use on or more AV synchrony metrics, in accordance with embodiments of the present technology. In FIG. 14, the system is a distributed processing system 1400 that includes a server 1402 connected to a database 1404, a programmer 1406, a local monitoring device 1408, and a user workstation 1410 electrically connected to a network 1412. Any one or more of the processor-based components, e.g., workstation 1410, cell phone 1414, local monitoring device 1416, server 1402, or programmer 1406, but not limited thereto, may perform the processes discussed herein.

The network 1412 may provide cloud-based services over the internet, a voice over IP (VoIP) gateway, a local plain old telephone service (POTS), a public switched telephone network (PSTN), a cellular phone-based network, and the like. Alternatively, the communication system may be a local area network (LAN), a medical campus area network (CAN), a metropolitan area network (MAN), or a wide area network (WAM). The communication system serves to provide a network that facilitates the transfer/receipt of data and other information between local and remote devices (relative to a patient). The server 1402 is a computer system that includes one or more processors and provides services to the other computing devices on the network 1412. The server 1402 controls the communication of information, such as physiologic signal segments, bradycardia episode information, asystole episode information, arrythmia episode information, markers, heart rates, and device settings. The server 1402 interfaces with the network 1412 to transfer information between the programmer 1406, local monitoring devices 1408, 1416, user workstation 1410, cell phone 1414 and database 1404. The database 1404 stores information, such as physiologic signal segments, arrythmia episode information, arrythmia statistics, diagnostics, heart rates, device settings, and the like, for a patient population, as well as separated for individual patients, individual physicians, individual clinics, individual medical networks and the like. The programmer 1406 may reside in a patient's home, a hospital, or a physician's office. The programmer 1406 may wirelessly communicate with IMD(s) 1403 (e.g., aLP 102 and/or vLP 104) and utilize protocols, such as Bluetooth, GSM, infrared wireless LANs, HIPERLAN, 3G, satellite, as well as circuit and packet data protocols, and the like. Alternatively, a telemetry "wand" connection may be used to connect the programmer 1406 to the IMD(s) 1403. The programmer 1406 is, e.g., able to acquire ECG signal segments from surface electrodes on a person, EGM signal segments from the IMD(s) 1403, and/or arrythmia episode information, arrythmia statistics, diagnostics, markers, atrial heart rates, device settings from the IMD(S) 1403. The programmer 1406 interfaces with the network 1412, either via the internet, to upload the information acquired from the surface ECG unit 1420, or the IMD(s) 1403 to the server 1402. The IMD(s) 1403 can be, e.g., the IMD 610 described above with reference to FIGS. 6 and 7, but is not limited thereto.

The local monitoring device 1408 interfaces with the communication system to upload to the server 1402 one or more physiologic signal segments, motion data, arrythmia episode information, arrythmia statistics, diagnostics, markers, heart rates, sensitivity profile parameter settings and detection thresholds. In one embodiment, the surface ECG unit 1420 and the IMD(s) 1403 have a bi-directional connection 1424 with the local RF monitoring device 1408 via a wireless connection. The local monitoring device 1408 is able to acquire surface ECG signal segments from one or more ECG leads 1422, as well as other information from the IMD(s) 1403. On the other hand, the local monitoring device 1408 may download the data and information discussed herein from the database 1404 to the IMD(s) 1403. It would also be possible for pulse oximeter sensor that obtains PPG segments to be communicatively coupled to one of the local monitoring devices 1408, 1416, or the programmer 1406, or a cell phone 1414.

The user workstation 1410, cell phone 1414 and/or programmer 1406 may be utilized by a physician or medical personnel to interface with the network 1412 to download physiologic signal segments, motion data, and other information discussed herein from the database 1404, from the local monitoring devices 1408, 1416, from the IMD(s) 1403 or otherwise. Once downloaded, the user workstation 1410 may process the physiologic signal segments and cause the display of portions thereof in accordance with one or more of the operations described above. The user workstation 1410, cell phone 1414 and/or programmer 1406, may be used to display portions of physiologic signal segments to a clinician, in accordance with embodiments of the present technology described herein.

The user workstation 1410, cell phone 1414 and/or programmer 1406 may upload/push settings, IMD instructions, other information and notifications to the cell phone 1414, local monitoring devices 1408, 1416, programmer 1406, server 1402 and/or IMD(s) 1403. The user workstation 1410, cell phone 1414 and/or programmer 1406 can each include, or be communicatively coupled to, a display screen, so that portions of physiologic signal segments can be displayed on the display screen utilizing embodiments of the present technology. The user workstation 1410, cell phone 1414 and/or programmer 1406 can each include, or be communicatively coupled to, a printer so that portions of physiologic signal segments can be displayed on a printout (aka a printed report) utilizing embodiments of the present technology.

The system of FIG. 14 further comprises one or more processors configured to execute the specific executable instructions to perform the steps described above, e.g., with reference to FIGS. 6A, 6B and/or 6C.

FIG. 15 illustrates a system level diagram indicating potential devices and networks that utilize the methods and systems herein. For example, an IMD 1502 may be utilized to sense and store physical signal segments. The IMD 1502 may supply the physiologic signal segments to various local external devices, such as a tablet device 1504, a smart phone 1506, a bedside monitoring device 1508, a smart watch and the like. The devices 1504-1508 can include, or be communicatively coupled to, a display screen to enable physiologic signal segments, or portions thereof, to be displayed a clinician or the like. The devices can also include, or communicatively coupled to, a printer that can print physiologic signal segments, or portions thereof, for display and analysis by a clinician or the like.

The IMD 1502 may convey the physiologic signal segments over various types of wireless communications links to the devices 1504, 1506 and 1508. The IMD 1502 may utilize various communications protocols and be activated in various manners, such as conductive communication, Bluetooth, Bluetooth low energy, Wi-Fi or other wireless protocol. Additionally or alternatively, when a magnetic device 1510 is held next to the patient, the magnetic field from the magnetic device 1510 may activate the IMD 1502 to transmit the physiologic signal segments and other information, such as arrythmia data, to one or more of the devices 1504-1508.

An aspect of the embodiments relates to a method for use with a dual chamber leadless pacemaker (LP) system including an atrial leadless pacemaker (aLP) and a ventricular leadless pacemaker (vLP) that are configured to communicate with one another, wherein the aLP and the vLP are configured to collectively provide DDD operation when an atrial-to-ventricular (a2v) message transmitted by the aLP is successfully received by the vLP, and a ventricular-to-atrial (v2a) message transmitted by the vLP is successfully received by the aLP, during a cardiac cycle. The aLP and the vLP are configured to collectively provide at least one of VDD operation, DDI operation or VDI operation at least some times when at least one of an a2v message transmitted by the aLP is not successfully received by the vLP, and/or a v2a message transmitted by the vLP is not successfully received by the aLP, during a further cardiac cycle. The method comprises obtaining a2v throughput, v2a throughput, and event sequence information, for a period of time during which the aLP and the vLP were configured to collectively provide DDD operation. The method also comprises determining, based on the a2v throughput and the v2a throughput, an estimated DDD metric, an estimated VDD metric, an estimated DDI metric, and an estimated VDI metric, for the period of time. The method additionally includes determining an atrio-ventricular (AV) synchrony metric for the period of time based on the estimated DDD metric, the estimated VDD metric, the estimated DDI metric, the estimated VDI metric, and the event sequence information, wherein the AV synchrony metric specifies an estimate of how often AV synchrony was achieved during the period of time.

In an embodiment, the method further comprises adjusting, based on the AV synchrony metric, at least one parameter used by the aLP for transmitting further a2v messages to the vLP, at least one parameter used by the vLP for receiving the further a2v messages from the aLP, at least one parameter used by the vLP for transmitting further v2a messages to the aLP, and/or at least one parameter used by the aLP for receiving the further v2a messages from the vLP.

In an embodiment, the method additionally or alternatively comprises adjusting, based on the AV synchrony metric, an implant location of at least one of the aLP or the vLP.

In an embodiment, the method additionally or alternatively comprises displaying the AV synchrony metric.

In an embodiment, the aLP is configured to transmit a2v messages to the vLP. The vLP is configured to attempt to receive the a2v messages from the aLP. The vLP is configured to transmit v2a messages to the aLP. The aLP is configured to attempt to receive the v2a messages from the vLP.

In an embodiment, the a2v throughput specifies how often a2v messages transmitted by the aLP were successfully received by the vLP during the period of time during which the aLP and the vLP were configured to collectively provide the DDD operation. The v2a throughput specifies how often v2a messages transmitted by the vLP were successfully received by the aLP during the period of time. The event sequence information specifies how often each of a plurality of different combinations of pacing and sensing occurred during the period of time.

In an embodiment, the estimated DDD metric specifies an estimate of how often the aLP and the vLP collectively provided the DDD operation during the period of time during which the aLP and the vLP were configured to collectively provide the DDD operation. The estimated VDD metric specifies an estimate of how often the aLP and the vLP collectively provided the VDD operation during the period of time. The estimated DDI metric specifies an estimate of how often the aLP and the vLP collectively provided the DDI operation during the period of time. The estimated VDI metric specifies an estimate of how often the aLP and the vLP collectively provided the VDI operation during the period of time.

In an embodiment, the event sequence information includes AP-VP information, AP-VS information, AS-VP information, and AS-VS information. The AP-VP information specifies how often the aLP provided atrial pacing (AP) and the vLP provided ventricular pacing (VP) for a same cardiac cycle within the period of time during which the aLP and the vLP were configured to collectively provide the DDD operation. The AP-VS information specifies how often the aLP provided AP and the vLP provided ventricular sensing (VS) for a same cardiac cycle within the period of time, the AS-VP information specifies how often the aLP provided atrial sensing (AS) and the vLP provided VP for a same cardiac cycle within the period of time. The AS-VS information specifies how often the aLP provided AS and the vLP provided VS for a same cardiac cycle within the period of time.

In an embodiment, the determining, based on the a2v throughput and the v2a throughput, the estimated DDD metric, the estimated VDD metric, the estimated DDI metric, and the estimated VDI metric, comprises: determining the estimated DDD metric is equal to a minimum of the a2v throughput and the v2a throughput; determining the estimated VDD metric is equal to the a2v throughput minus the estimated DDD metric; determining the estimated DDI metric is equal to the v2a throughput minus the estimated DDD metric; and determining the estimated VDI metric is equal to 100% minus a sum of the estimated DDD metric, the estimated VDD metric, and the estimated DDI metric.

In an embodiment, the determining, based on the a2v throughput and the v2a throughput, the estimated DDD metric, the estimated VDD metric, the estimated DDI metric, and the estimated VDI metric, comprises: determining the estimated DDD metric is equal to a maximum of zero and 100% minus a sum of a percentage of a2v messages that were not successfully received by the vLP and a percentage of v2a messages that were not successfully received by the aLP; determining the estimated VDD metric is equal to the a2v throughput minus the estimated DDD metric; determining the estimated DDI metric is equal to the v2a throughput minus the estimated DDD metric; and determining the estimated VDI metric is equal to 100% minus a sum of the estimated DDD metric, the estimated VDD metric, and the estimated DDI metric.

In an embodiment, the method is performed by a non-implanted system that receives the a2v throughput and the v2a throughput from at least one of the aLP or the vLP, or receives the a2v throughput and the v2a throughput from another system that had received the a2v throughput and the v2a throughput from at least one of the aLP or the vLP.

In an embodiment, the method is performed by at least one of the aLP or the vLP.

Another aspect of the embodiments relates to a system including, or for use with, an atrial leadless pacemaker (aLP) and a ventricular leadless pacemaker (vLP) that are configured to communicate with one another, wherein the aLP and the vLP are configured to collectively provide DDD operation when an atrial-to-ventricular (a2v) message transmitted by the aLP is successfully received by the vLP, and a ventricular-to-atrial (v2a) message transmitted by the vLP is successfully received by the aLP, during a cardiac cycle. The aLP and the vLP are configured to collectively provide at least one of VDD operation, DDI operation or VDI operation at least some times when at least one of an a2v message transmitted by the aLP is not successfully received by the vLP, and/or a v2a message transmitted by the vLP is not successfully received by the aLP, during a further cardiac cycle. The system comprises one or more processors configured to obtain a2v throughput, v2a throughput, and event sequence information, for a period of time during which the aLP and the vLP were configured to collectively provide the DDD operation. The one or more processors of the system is/are also configured to determine, based on the a2v throughput and the v2a throughput, an estimated DDD metric, an estimated VDD metric, an estimated DDI metric, and an estimated VDI metric, for the period of time. The one or more processors of the system is/are also configured to determine an atrio-ventricular (AV) synchrony metric for the period of time based on the estimated DDD metric, the estimated VDD metric, the estimated DDI metric, the estimated VDI metric, and the event sequence information, wherein the AV synchrony metric specifies an estimate of how often AV synchrony was achieved during the period of time.

In an embodiment, the system includes the aLP and the vLP, each of which includes a respective processor and a respective at least two electrodes, wherein the aLP is configured to be implanted in or on an atrial cardiac chamber and the vLP is configured to be implanted in or on a ventricular cardiac chamber. Additionally, the aLP is configured to transmit a2v messages to the vLP, the vLP is configured to attempt to receive the a2v messages from the aLP. The vLP is configured to transmit v2a messages to the aLP, and the aLP is configured to attempt to receive the v2a messages from the vLP.

In an embodiment, the processor of the vLP or the processor of the aLP comprises at least one of the one or more processors configured to determine the AV synchrony metric. Additionally, at least one of the processor of the vLP or the processor of the aLP is further configured to adjust one or more parameters of at least one of the aLP or the vLP based on the AV synchrony metric.

In an embodiment, the system includes a non-implanted subsystem (e.g., device) that includes at least one processor, of the one or more processors, that is/are configured to determine the AV synchrony metric for the period of time. The at least one processor of the non-implanted subsystem (e.g., device) is configured to adjust one or more parameters of at least one of the aLP or the vLP based on the AV synchrony metric.

In an embodiment, the one or more processors that is/are configured to determine the AV synchrony metric for the period of time, is/are also configured to adjust, based on the AV synchrony metric, at least one parameter used by the aLP for transmitting further a2v messages to the vLP, at least one parameter used by the vLP for receiving the further a2v messages from the aLP, at least one parameter used by the vLP for transmitting further v2a messages to the aLP, and/or at least one parameter used by the aLP for receiving the further v2a messages from the vLP.

In an embodiment, the one or more processors that is/are configured to determine the AV synchrony metric for the period of time, is/are also configured to cause the AV synchrony metric to be displayed.

In an embodiment, the one or more processors that is/are configured to determine the AV synchrony metric for the period of time, is/are also configured to provide a notification that an implant location of at least one of the aLP or the vLP should be modified.

In an embodiment, the estimated DDD metric specifies an estimate of how often the aLP and the vLP collectively provided the DDD operation during the period of time during which the aLP and the vLP were configured to collectively provide the DDD operation. The estimated VDD metric specifies an estimate of how often the aLP and the vLP collectively provided the VDD operation during the period of time. The estimated DDI metric specifies an estimate of how often the aLP and the vLP collectively provided the DDI operation during the period of time. The estimated VDI metric specifies an estimate of how the aLP and the vLP collectively provided the VDI operation during the period of time.

In an embodiment, the event sequence information includes AP-VP information, AP-VS information, AS-VP information, and AS-VS information. The AP-VP information specifies how often the aLP provided atrial pacing (AP) and the vLP provided ventricular pacing (VP) for a same cardiac cycle within the period of time during which the aLP and the vLP were configured to collectively provide the DDD operation. The AP-VS information specifies how often the aLP provided AP and the vLP provided ventricular sensing (VS) for a same cardiac cycle within the period of time. The AS-VP information specifies how often the aLP provided atrial sensing (AS) and the vLP provided VP for a same cardiac cycle within the period of time. The AS-VS information specifies how often the aLP provided AS and the vLP provided VS for a same cardiac cycle within the period of time.

Another aspect of the embodiments relates to a method for use with a dual chamber leadless pacemaker (LP) system including an atrial leadless pacemaker (aLP) and a ventricular leadless pacemaker (vLP) that are configured to communicate with one another and to collectively provide DDD operation when an atrial-to-ventricular (a2v) message transmitted by the aLP is successfully received by the vLP and a ventricular-to-atrial (v2a) message transmitted by the vLP is successfully received by the aLP during a cardiac cycle, and wherein the aLP and the vLP are configured to collectively provide at least one of VDD, DDI or VDI operation at least some times when at least one of an a2v message transmitted by the aLP is not successfully received by the vLP and/or a v2a message transmitted by the vLP is not successfully received by the aLP during a further cardiac cycle. The method comprising: obtaining a2v throughput, v2a throughput, and event sequence information, for a period of time during which the aLP and the vLP were configured to collectively provide DDD operation; determining, based on the a2v throughput and the v2a throughput, estimates of how often the aLP and the vLP collectively provide each of DDD, VDD, DDI, and VVI operation during the period of time; determining an atrio-ventricular (AV) synchrony metric for the period of time based on the estimates; and adjusting, based on the AV synchrony metric, at least one parameter used by the aLP for communicating with the vLP and/or at least one parameter used by the vLP for communicating with the aLP. In accordance with certain embodiments, the method is performed by a non-implanted system that receives the a2v throughput and the v2a throughput from at least one of the aLP or the vLP, or receives the a2v throughput and the v2a throughput from another system that had received the a2v throughput and the v2a throughput from at least one of the aLP or the vLP. In accordance with other embodiments, the method is performed by at least one of the aLP or the vLP.

Another aspect of the embodiments relates to a system including or for use with an atrial leadless pacemaker (aLP) and a ventricular leadless pacemaker (vLP) that are configured to communicate with one another and collectively provide DDD operation when an atrial-to-ventricular (a2v) message transmitted by the aLP is successfully received by the vLP and a ventricular-to-atrial (v2a) message transmitted by the vLP is successfully received by the aLP during a cardiac cycle, and wherein the aLP and the vLP are configured to collectively provide at least one of VDD, DDI or VDI operation at least some times when at least one of an a2v message transmitted by the aLP is not successfully received by the vLP and/or a v2a message transmitted by the vLP is not successfully received by the aLP during a further cardiac cycle, the system comprising one or more processors configured to: obtain a2v throughput, v2a throughput, and event sequence information, for a period of time during which the aLP and the vLP were configured to collectively provide DDD operation; determine, based on the a2v throughput and the v2a throughput, estimates of how often the aLP and the vLP collectively provide each of DDD, VDD, DDI, and VVI operation during the period of time; determine an atrio-ventricular (AV) synchrony metric for the period of time based on the estimates; and adjust, based on the AV synchrony metric, at least one parameter used by the aLP for communicating with the vLP and/or at least one parameter used by the vLP for communicating with the aLP. In accordance with certain embodiments, the system includes the aLP and the vLP, each of which includes a respective processor and a respective at least two electrodes; at least one of the processor of the vLP or the processor of the aLP comprises at least one of the one or more processors configured to determine the AV synchrony metric; and at least one of the processor of the vLP or the processor of the aLP is configured to adjust at least one parameter used by the aLP for communicating with the vLP and/or at least one parameter used by the vLP for communicating with the aLP. In accordance with other embodiments, the system includes a non-implanted subsystem that includes at least one processor, of the one or more processors, that is/are configured to determine the AV synchrony metric for the period of time and to adjust, based on the AV synchrony metric, at least one parameter used by the aLP for communicating with the vLP and/or at least one parameter used by the vLP for communicating with the aLP.

A method for use with a dual chamber leadless pacemaker (LP) system including an atrial leadless pacemaker (aLP) and a ventricular leadless pacemaker (vLP) that are configured to communicate with one another is disclosed. The aLP and the vLP are configured to collectively provide DDD operation when an atrial-to-ventricular (a2v) message transmitted by the aLP is successfully received by the vLP, and a ventricular-to-atrial (v2a) message transmitted by the vLP is successfully received by the aLP, during a cardiac cycle. The aLP and the vLP are configured to collectively provide at least one of VDD operation, DDI operation or VDI operation at least some times when at least one of an a2v message transmitted by the aLP is not successfully received by the vLP, and/or a v2a message transmitted by the vLP is not successfully received by the aLP, during a further cardiac cycle. The method comprises obtaining a2v throughput, v2a throughput, and event sequence information, for a period of time during which the aLP and the vLP were configured to collectively provide DDD operation. The method also comprises determining, based on the a2v throughput and the v2a throughput, an estimated DDD metric, an estimated VDD metric, an estimated DDI metric, and an estimated VDI metric, for the period of time. The method additionally includes determining an atrio-ventricular (AV) synchrony metric for the period of time based on the estimated DDD metric, the estimated VDD metric, the estimated DDI metric, the estimated VDI metric, and the event sequence information, wherein the AV synchrony metric specifies an estimate of how often AV synchrony was achieved during the period of time.

Optionally, the method further comprises adjusting, based on the AV synchrony metric, at least one parameter used by the aLP for transmitting further a2v messages to the vLP, at least one parameter used by the vLP for receiving the further a2v messages from the aLP, at least one parameter used by the vLP for transmitting further v2a messages to the aLP, and/or at least one parameter used by the aLP for receiving the further v2a messages from the vLP.

Optionally, the method additionally or alternatively comprises adjusting, based on the AV synchrony metric, an implant location of at least one of the aLP or the vLP.

Optionally, the method additionally or alternatively comprises displaying the AV synchrony metric.

Optionally, the aLP is configured to transmit a2v messages to the vLP. The vLP is configured to attempt to receive the a2v messages from the aLP. The vLP is configured to transmit v2a messages to the aLP. The aLP is configured to attempt to receive the v2a messages from the vLP.

Optionally, the a2v throughput specifies how often a2v messages transmitted by the aLP were successfully received by the vLP during the period of time during which the aLP and the vLP were configured to collectively provide the DDD operation. The v2a throughput specifies how often v2a messages transmitted by the vLP were successfully received by the aLP during the period of time. The event sequence information specifies how often each of a plurality of different combinations of pacing and sensing occurred during the period of time.

Optionally, the estimated DDD metric specifies an estimate of how often the aLP and the vLP collectively provided the DDD operation during the period of time during which the aLP and the vLP were configured to collectively provide the DDD operation. The estimated VDD metric specifies an estimate of how often the aLP and the vLP collectively provided the VDD operation during the period of time. The estimated DDI metric specifies an estimate of how often the aLP and the vLP collectively provided the DDI operation during the period of time. The estimated VDI metric specifies an estimate of how often the aLP and the vLP collectively provided the VDI operation during the period of time.

Optionally, the event sequence information includes AP-VP information, AP-VS information, AS-VP information, and AS-VS information. The AP-VP information specifies how often the aLP provided atrial pacing (AP) and the vLP provided ventricular pacing (VP) for a same cardiac cycle within the period of time during which the aLP and the vLP were configured to collectively provide the DDD operation. The AP-VS information specifies how often the aLP provided AP and the vLP provided ventricular sensing (VS) for a same cardiac cycle within the period of time, the AS-VP information specifies how often the aLP provided atrial sensing (AS) and the vLP provided VP for a same cardiac cycle within the period of time. The AS-VS information specifies how often the aLP provided AS and the vLP provided VS for a same cardiac cycle within the period of time.

Optionally, the determining, based on the a2v throughput and the v2a throughput, the estimated DDD metric, the estimated VDD metric, the estimated DDI metric, and the estimated VDI metric, comprises: determining the estimated DDD metric is equal to a minimum of the a2v throughput and the v2a throughput; determining the estimated VDD metric is equal to the a2v throughput minus the estimated DDD metric; determining the estimated DDI metric is equal to the v2a throughput minus the estimated DDD metric; and determining the estimated VDI metric is equal to 100% minus a sum of the estimated DDD metric, the estimated VDD metric, and the estimated DDI metric.

Optionally, the determining, based on the a2v throughput and the v2a throughput, the estimated DDD metric, the estimated VDD metric, the estimated DDI metric, and the estimated VDI metric, comprises: determining the estimated DDD metric is equal to a maximum of zero and 100% minus a sum of a percentage of a2v messages that were not successfully received by the vLP and a percentage of v2a messages that were not successfully received by the aLP; determining the estimated VDD metric is equal to the a2v throughput minus the estimated DDD metric; determining the estimated DDI metric is equal to the v2a throughput minus the estimated DDD metric; and determining the estimated VDI metric is equal to 100% minus a sum of the estimated DDD metric, the estimated VDD metric, and the estimated DDI metric.

Optionally, the method is performed by a non-implanted system that receives the a2v throughput and the v2a throughput from at least one of the aLP or the vLP, or receives the a2v throughput and the v2a throughput from another system that had received the a2v throughput and the v2a throughput from at least one of the aLP or the vLP.

Optionally, the method is performed by at least one of the aLP or the vLP.

A method for use with a dual chamber leadless pacemaker (LP) system including an atrial leadless pacemaker (aLP) and a ventricular leadless pacemaker (vLP) that are configured to communicate with one another and to collectively provide DDD operation when an atrial-to-ventricular (a2v) message transmitted by the aLP is successfully received by the vLP and a ventricular-to-atrial (v2a) message transmitted by the vLP is successfully received by the aLP during a cardiac cycle, and wherein the aLP and the vLP are configured to collectively provide at least one of VDD, DDI or VDI operation at least some times when at least one of an a2v message transmitted by the aLP is not successfully received by the vLP and/or a v2a message transmitted by the vLP is not successfully received by the aLP during a further cardiac cycle. The method comprising: obtaining a2v throughput, v2a throughput, and event sequence information, for a period of time during which the aLP and the vLP were configured to collectively provide DDD operation; determining, based on the a2v throughput and the v2a throughput, estimates of how often the aLP and the vLP collectively provide each of DDD, VDD, DDI, and VVI operation during the period of time; determining an atrio-ventricular (AV) synchrony metric for the period of time based on the estimates; and adjusting, based on the AV synchrony metric, at least one parameter used by the aLP for communicating with the vLP and/or at least one parameter used by the vLP for communicating with the aLP. In accordance with certain embodiments, the method is performed by a non-implanted system that receives the a2v throughput and the v2a throughput from at least one of the aLP or the vLP, or receives the a2v throughput and the v2a throughput from another system that had received the a2v throughput and the v2a throughput from at least one of the aLP or the vLP. In accordance with other embodiments, the method is performed by at least one of the aLP or the vLP.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, it is noted that the term "based on" as used herein, unless stated otherwise, should be interpreted as meaning based at least in part on, meaning there can be one or more additional factors upon which a decision or the like is made. For example, if a decision is based on the results of a comparison, that decision can also be based on one or more other factors in addition to being based on results of the comparison.

Embodiments of the present technology have been described above with the aid of functional building blocks illustrating the performance of specified functions and relationships thereof. The boundaries of these functional building blocks have often been defined herein for the convenience of the description. Alternate boundaries can be defined so long as the specified functions and relationships thereof are appropriately provided. Any such alternate boundaries are thus within the scope and spirit of the claimed invention. For example, it would be possible to combine or separate some of the steps shown in FIGS. 6A, 6B and 6C. For another example, it is possible to change the boundaries of some of the dashed blocks shown in FIGS. 1B, 13, 14, and 15. For example, step 602 can be divided into three sub-steps, and step 604 can be divided into four sub-steps.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the embodiments of the present technology without departing from its scope. While the dimensions, types of materials and coatings described herein are intended to define the parameters of the embodiments of the present technology, they are by no means limiting and are exemplary embodiments. Many other embodiments will be apparent to those of skill in the art upon reviewing the above description. The scope of the embodiments of the present technology should, therefore, be determined with reference to the appended claims. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. A system (100) including, or for use with, an atrial leadless pacemaker, aLP, (102) and a ventricular leadless pacemaker, vLP, (104) that are configured to communicate with one another,
wherein the aLP (102) and the vLP (104) are configured to collectively provide DDD operation when an atrial-to-ventricular, a2v, message transmitted by the aLP (102) is successfully received by the vLP (104), and a ventricular-to-atrial, v2a, message transmitted by the vLP (104) is successfully received by the aLP (102), during a cardiac cycle, and
wherein the aLP (102) and the vLP (104) are configured to collectively provide at least one of VDD operation, DDI operation or VDI operation at least some times when at least one of an a2v message transmitted by the aLP (102) is not successfully received by the vLP (104), and/or a v2a message transmitted by the vLP (104) is not successfully received by the aLP (102), during a further cardiac cycle,
the system (100) comprising one or more processors (112, 1320), **characterized in that** the one or more processors (112, 1320) is/are configured to:
obtain a2v throughput, v2a throughput, and event sequence information, for a period of time during which the aLP (102) and the vLP (104) were configured to collectively provide the DDD operation, wherein the a2v throughput specifies how often a2v messages transmitted by the aLP (102) were successfully received by the vLP (104) during the period of time during which the aLP (102) and the vLP (104) were configured to collectively provide the DDD operation, and wherein the v2a throughput specifies how often v2a messages transmitted by the vLP (104) were successfully received by the aLP (102) during the period of time;
determine, based on the a2v throughput and the v2a throughput, an estimated DDD metric, an estimated VDD metric, an estimated DDI metric, and an estimated VDI metric, for the period of time; and
determine an atrio-ventricular, AV, synchrony metric for the period of time based on the estimated DDD metric, the estimated VDD metric, the estimated DDI metric, the estimated VDI metric, and the event sequence information, wherein the AV synchrony metric specifies an estimate of how often AV synchrony was achieved during the period of time.

2. The system (100) of claim 1, wherein
the system (100) includes the aLP (102) and the vLP (104), each of which includes a respective processor of the one or more processors (112, 1320), and each of which includes a respective at least two electrodes (108);
the aLP (102) is configured to be implanted in or on an atrial cardiac chamber;
the vLP (104) is configured to be implanted in or on a ventricular cardiac chamber; and
the aLP (102) is configured to transmit a2v messages to the vLP (104), the vLP (104) is configured to attempt to receive the a2v messages from the aLP (102), the vLP (104) is configured to transmit v2a messages to the aLP (102), and the aLP (102) is configured to attempt to receive the v2a messages from the vLP (104).

3. The system (100) of claim 2, wherein
the processor (112, 1320) of the vLP (104) or the processor (112, 1320) of the aLP (102) comprises at least one of the one or more processors (112, 1320) configured to determine the AV synchrony metric; and
at least one of the processor (112, 1320) of the vLP (104) or the processor (112, 1320) of the aLP (102) is further configured to adjust one or more parameters of at least one of the aLP (102) or the vLP (104) based on the AV synchrony metric.

4. The system (100) of any one of claims 1 through 3, wherein
the system (100) includes a non-implanted subsystem (109) that includes at least one processor (112, 1320), of the one or more processors (112, 1320), that is/are configured to determine the AV synchrony metric for the period of time; and
the at least one processor (112, 1320) of the non-implanted subsystem (109) is configured to adjust one or more parameters of at least one of the aLP (102) or the vLP (104) based on the AV synchrony metric.

5. The system of claim 4, wherein the aLP (102) and the vLP (104) are configured to communicate with the non-implanted subsystem (109) using conducted communication.

6. The system of claim 4 or 5, wherein the non-implanted subsystem (109) comprises a programmer that is configured to communicate with the aLP (102) and the vLP (104).

7. The system of any one of claims 4 through 6, wherein the non-implanted subsystem (109) comprises one of a tablet device (1504), a smart phone (1506), or a smart watch, that is configured to communicate with the aLP (102) and the vLP (104).

8. The system of any one of claims 4 through 7, wherein the non-implanted subsystem (109) comprises one of a bedside monitor (1508), a patient link module, or a remote patient care network.

9. The system (100) of any one of claims 1 through 8, wherein the one or more processors (112, 1320) that is/are configured to determine the AV synchrony metric for the period of time, is also configured to adjust, based on the AV synchrony metric, at least one parameter used by the aLP (102) for transmitting further a2v messages to the vLP (104), at least one parameter used by the vLP (104) for receiving the further a2v messages from the aLP (102), at least one parameter used by the vLP (104) for transmitting further v2a messages to the aLP (102), and/or at least one parameter used by the aLP (102) for receiving the further v2a messages from the vLP (104).

10. The system (100) of any one of claims 1 through 9, wherein the one or more processors (112, 1320) that is/are configured to determine the AV synchrony metric for the period of time, is/are also configured to cause the AV synchrony metric to be displayed.

11. The system (100) of any one of claims 1 through 10, wherein the one or more processors (112, 1320) that is/are configured to determine the AV synchrony metric for the period of time, is/are also configured to provide a notification that an implant location of at least one of the aLP (102) or the vLP (104) should be modified.

12. The system (100) of any one of claims 1 through 11, wherein the estimated DDD metric specifies an estimate of how often the aLP (102) and the vLP (104) collectively provided the DDD operation during the period of time during which the aLP (102) and the vLP (104) were configured to collectively provide the DDD operation;
the estimated VDD metric specifies an estimate of how often the aLP (102) and the vLP (104) collectively provided the VDD operation during the period of time;
the estimated DDI metric specifies an estimate of how often the aLP (102) and the vLP (104) collectively provided the DDI operation during the period of time; and
the estimated VDI metric specifies an estimate of how the aLP (102) and the vLP (104) collectively provided the VDI operation during the period of time.

13. The system (100) of any one of claims 1 through 12, wherein
the event sequence information includes AP-VP information, AP-VS information, AS-VP information, and AS-VS information;
the AP-VP information specifies how often the aLP (102) provided atrial pacing, AP, and the vLP (104) provided ventricular pacing, VP, for a same cardiac cycle within the period of time during which the aLP (102) and the vLP (104) were configured to collectively provide the DDD operation;
the AP-VS information specifies how often the aLP (102) provided AP and the vLP (104) provided ventricular sensing, VS, for a same cardiac cycle within the period of time;
the AS-VP information specifies how often the aLP (102) provided atrial sensing, AS, and the vLP (104) provided VP for a same cardiac cycle within the period of time; and
the AS-VS information specifies how often the aLP (102) provided AS and the vLP (104) provided VS for a same cardiac cycle within the period of time.

14. The system (100) of any one of claims 1 through 13, wherein the one or more processors (112, 1320) is/are configured to:
determine the estimated DDD metric is equal to a minimum of the a2v throughput and the v2a throughput, or is equal to a maximum of zero and 100% minus a sum of a percentage of a2v messages that were not successfully received by the vLP (104) and a percentage of v2a messages that were not successfully received by the aLP (102);
determine the estimated VDD metric is equal to the a2v throughput minus the estimated DDD metric;
determine the estimated DDI metric is equal to the v2a throughput minus the estimated DDD metric; and
determine the estimated VDI metric is equal to 100% minus a sum of the estimated DDD metric, the estimated VDD metric, and the estimated DDI metric.

15. The system of any one of claims 1 through 14, wherein the aLP (102) and the vLP (104) are configured to communicate with one another using conducted communication.

## Patentansprüche

1. System (100), beinhaltend einen kabellosen atrialen Herzschrittmacher, aLP, (102) und einen kabellosen ventrikulären Herzschrittmacher, vLP, (104) oder zur Verwendung mit diesen, die dazu konfiguriert sind, miteinander zu kommunizieren,
wobei der aLP (102) und der vLP (104) dazu konfiguriert sind, gemeinsam DDD-Betrieb bereitzustellen, wenn während eines Herzzyklus eine atrial-zu-ventrikuläre Nachricht, a2v-Nachricht, die durch den aLP (102) übertragen wird, erfolgreich durch den vLP (104) empfangen wird und eine ventrikulär-zuatriale Nachricht, v2a-Nachricht, die durch den vLP (104) übertragen wird, erfolgreich durch den aLP (102) empfangen wird, und
wobei der aLP (102) und der vLP (104) dazu konfiguriert sind, gemeinsam mindestens einen von VDD-Betrieb, DDI-Betrieb oder VDI-Betrieb mindestens einige Male bereitzustellen, wenn während eines weiteren Herzzyklus mindestens eine von einer a2v-Nachricht, die durch den aLP (102) übertragen wird, nicht erfolgreich durch den vLP (104) empfangen wird und/oder eine v2a-Nachricht, die durch den vLP (104) übertragen wird, nicht erfolgreich durch den aLP (102) empfangen wird,
wobei das System (100) einen oder mehrere Prozessoren (112, 1320) umfasst, **dadurch gekennzeichnet, dass** der eine oder die mehreren Prozessoren (112, 1320) zu Folgendem konfiguriert ist/sind:
Erhalten von a2v-Durchsatz, v2a-Durchsatz und Ereignisfolgeinformationen für eine Zeitspanne, während welcher der aLP (102) und der vLP (104) dazu konfiguriert waren, gemeinsam den DDD-Betrieb bereitzustellen, wobei der a2v-Durchsatz angibt, wie oft a2v-Nachrichten, die durch den aLP (102) übertragen wurden, erfolgreich durch den vLP (104) während der Zeitspanne empfangen wurden, während welcher der aLP (102) und der vLP (104) dazu konfiguriert waren, gemeinsam den DDD-Betrieb bereitzustellen, und wobei der v2a-Durchsatz angibt, wie oft v2a-Nachrichten, die durch den vLP (104) übertragen wurden, erfolgreich durch den aLP (102) während der Zeitspanne empfangen wurden;
Bestimmen, basierend auf dem a2v-Durchsatz und dem v2a-Durchsatz, einer geschätzten DDD-Metrik, einer geschätzten VDD-Metrik, einer geschätzten DDI-Metrik und einer geschätzten VDI-Metrik für die Zeitspanne; und
Bestimmen einer Metrik für eine atrio-ventrikuläre Synchronität, AV-Synchronitätsmetrik, für die Zeitspanne basierend auf der geschätzten DDD-Metrik, der geschätzten VDD-Metrik, der geschätzten DDI-Metrik, der geschätzten VDI-Metrik und der Ereignisfolgeinformationen, wobei die AV-Synchronitätsmetrik eine Schätzung darüber angibt, wie oft AV-Synchronität während der Zeitspanne erreicht wurde.

2. System (100) nach Anspruch 1, wobei
das System (100) den aLP (102) und den vLP (104) beinhaltet, von denen jeder einen jeweiligen Prozessor des einen oder der mehreren Prozessoren (112, 1320) beinhaltet und von denen jeder eine jeweilige mindestens zwei Elektroden (108) beinhaltet;
der aLP (102) dazu konfiguriert ist, in oder auf einer atrialen Herzkammer implantiert zu werden;
der vLP (104) dazu konfiguriert ist, in oder auf einer ventrikulären Herzkammer implantiert zu werden; und
der aLP (102) dazu konfiguriert ist, a2v-Nachrichten an den vLP (104) zu übertragen, der vLP (104) dazu konfiguriert ist, zu versuchen, die a2v-Nachrichten von dem aLP (102) zu empfangen, der vLP (104) dazu konfiguriert ist, v2a-Nachrichten an den aLP (102) zu übertragen, und der aLP (102) dazu konfiguriert ist, zu versuchen, die v2a-Nachrichten von dem vLP (104) zu empfangen.

3. System (100) nach Anspruch 2, wobei
der Prozessor (112, 1320) des vLP (104) oder der Prozessor (112, 1320) des aLP (102) mindestens einen von dem einen oder den mehreren Prozessoren (112, 1320) umfasst, die dazu konfiguriert sind, die AV-Synchronitätsmetrik zu bestimmen; und
mindestens einer von dem Prozessor (112, 1320) des vLP (104) oder des Prozessors (112, 1320) des aLP (102) ferner dazu konfiguriert ist, einen oder mehrere Parameter von mindestens einem von dem aLP (102) oder dem vLP (104) basierend auf der AV-Synchronitätsmetrik anzupassen.

4. System (100) nach einem der Ansprüche 1 bis 3, wobei
das System (100) ein nicht-implantiertes Teilsystem (109) beinhaltet, das mindestens einen Prozessor (112, 1320) des einen oder der mehreren Prozessoren (112, 1320) beinhaltet, der/die dazu konfiguriert ist/sind, die AV-Synchronitätsmetrik für die Zeitspanne zu bestimmen; und
der mindestens eine Prozessor (112, 1320) des nicht-implantierten Teilsystems (109) dazu konfiguriert ist, einen oder mehrere Parameter von mindestens einem von dem aLP (102) oder dem vLP (104) basierend auf der AV-Synchronitätsmetrik anzupassen.

5. System nach Anspruch 4, wobei der aLP (102) und der vLP (104) dazu konfiguriert sind, mit dem nicht-implantierten Teilsystem (109) unter Verwendung von geführter Kommunikation zu kommunizieren.

6. System nach Anspruch 4 oder 5, wobei das nicht-implantierte Teilsystem (109) einen Programmierer umfasst, der dazu konfiguriert ist, mit dem aLP (102) und dem vLP (104) zu kommunizieren.

7. System nach einem der Ansprüche 4 bis 6, wobei das nicht-implantierte Teilsystem (109) eines von einer Tablet-Vorrichtung (1504), einem Smartphone (1506) oder einer Smartwatch umfasst, die/das dazu konfiguriert ist, mit dem aLP (102) und dem vLP (104) zu kommunizieren.

8. System nach einem der Ansprüche 4 bis 7, wobei das nicht-implantierte Teilsystem (109) eines von einer bettseitigen Überwachungseinrichtung (1508), einem Patientenverbindungsmodul oder einem Netzwerk für die Fernbehandlung von Patienten umfasst.

9. System (100) nach einem der Ansprüche 1 bis 8, wobei der eine oder die mehreren Prozessoren (112, 1320), der/die dazu konfiguriert ist/sind, die AV-Synchronitätsmetrik für die Zeitspanne zu bestimmen, auch dazu konfiguriert ist, basierend auf der AV-Synchronitätsmetrik mindestens einen Parameter, der durch den aLP (102) zum Übertragen von weiteren a2v-Nachrichten an den vLP (104) verwendet wird, mindestens einen Parameter, der durch den vLP (104) zum Empfangen der weiteren a2v-Nachrichten von dem aLP (102) verwendet wird, mindestens einen Parameter, der durch den vLP (104) zum Übertragen von weiteren v2a-Nachrichten an den aLP (102) verwendet wird, und/oder mindestens einen Parameter, der durch den aLP (102) zum Empfangen der weiteren v2a-Nachrichten von dem vLP (104) verwendet wird, anzupassen.

10. System (100) nach einem der Ansprüche 1 bis 9, wobei der eine oder die mehreren Prozessoren (112, 1320), der/die dazu konfiguriert ist/sind, die AV-Synchronitätsmetrik für die Zeitspanne zu bestimmen, auch dazu konfiguriert ist/sind, die AV-Synchronitätsmetrik dazu zu veranlassen, angezeigt zu werden.

11. System (100) nach einem der Ansprüche 1 bis 10, wobei der eine oder die mehreren Prozessoren (112, 1320), der/die dazu konfiguriert ist/sind, die AV-Synchronitätsmetrik für die Zeitspanne zu bestimmen, auch dazu konfiguriert ist/sind, eine Benachrichtigung bereitzustellen, dass eine Implantatposition von mindestens einem von dem aLP (102) oder dem vLP (104) modifiziert werden sollte.

12. System (100) nach einem der Ansprüche 1 bis 11, wobei die geschätzte DDD-Metrik eine Schätzung darüber angibt, wie oft der aLP (102) und der vLP (104) gemeinsam den DDD-Betrieb während der Zeitspanne, während welcher der aLP (102) und der vLP (104) dazu konfiguriert waren, gemeinsam den DDD-Betrieb bereitzustellen, bereitgestellt haben;
die geschätzte VDD-Metrik eine Schätzung darüber angibt, wie oft der aLP (102) und der vLP (104) gemeinsam den VDD-Betrieb während der Zeitspanne bereitgestellt haben;
die geschätzte DDI-Metrik eine Schätzung darüber angibt, wie oft der aLP (102) und der vLP (104) gemeinsam den DDI-Betrieb während der Zeitspanne bereitgestellt haben; und
die geschätzte VDI-Metrik eine Schätzung darüber angibt, wie der aLP (102) und der vLP (104) gemeinsam den VDI-Betrieb während der Zeitspanne bereitgestellt haben.

13. System (100) nach einem der Ansprüche 1 bis 12, wobei
die Ereignisfolgeinformationen AP-VP-Informationen, AP-VS-Informationen, AS-VP-Informationen und AS-VS-Informationen beinhalten;
die AP-VP-Informationen angeben, wie oft für einen gleichen Herzzyklus innerhalb der Zeitspanne, während welcher der aLP (102) und der vLP (104) dazu konfiguriert waren, gemeinsam den DDD-Betrieb bereitzustellen, der aLP (102) atriale Stimulation, AP, bereitgestellt hat und der vLP (104) ventrikuläre Stimulation, VP, bereitgestellt hat;
die AP-VS-Informationen angeben, wie oft für einen gleichen Herzzyklus innerhalb der Zeitspanne der aLP (102) AP bereitgestellt hat und der vLP (104) ventrikuläres Abtasten, VS, bereitgestellt hat;
die AS-VP-Informationen angeben, wie oft für einen gleichen Herzzyklus innerhalb der Zeitspanne der aLP (102) atriales Abtasten, AS, bereitgestellt hat und der vLP (104) VP bereitgestellt hat; und
die AS-VS-Informationen angeben, wie oft für einen gleichen Herzzyklus innerhalb der Zeitspanne der aLP (102) AS bereitgestellt hat und der vLP (104) VS bereitgestellt hat.

14. System (100) nach einem der Ansprüche 1 bis 13, wobei der eine oder die mehreren Prozessoren (112, 1320) zu Folgendem konfiguriert ist/sind:
Bestimmen, dass die geschätzte DDD-Metrik gleich einem Minimum des a2v-Durchsatzes und des v2a-Durchsatzes ist oder gleich einem Maximum von Null und 100 % abzüglich einer Summe eines Prozentsatzes von a2v-Nachrichten, die nicht erfolgreich durch den vLP (104) empfangen wurden, und eines Prozentsatzes von v2a-Nachrichten, die nicht erfolgreich durch den aLP (102) empfangen wurden, ist;
Bestimmen, dass die geschätzte VDD-Metrik gleich dem a2v-Durchsatz minus der geschätzten DDD-Metrik ist;
Bestimmen, dass die geschätzte DDI-Metrik gleich dem v2a-Durchsatz minus der geschätzten DDD-Metrik ist; und
Bestimmen, dass die geschätzte VDI-Metrik gleich 100 % abzüglich einer Summe der geschätzten DDD-Metrik, der geschätzten VDD-Metrik und der geschätzten DDI-Metrik ist.

15. System nach einem der Ansprüche 1 bis 14, wobei der aLP (102) und der vLP (104) dazu konfiguriert sind, miteinander unter Verwendung von geführter Kommunikation zu kommunizieren.

## Revendications

1. Système (100) comprenant, ou destiné à être utilisé avec, un stimulateur cardiaque auriculaire sans fil, aLP, (102) et un stimulateur cardiaque ventriculaire sans fil, vLP, (104) configurés pour communiquer entre eux,
dans lequel l'aLP (102) et le vLP (104) sont configurés pour assurer collectivement un fonctionnement DDD lorsqu'un message auriculaire-ventriculaire, a2v, transmis par l'aLP (102) est reçu par le vLP (104) et qu'un message ventriculaire-auriculaire, v2a, transmis par le vLP (104) est reçu par l'aLP (102), pendant un cycle cardiaque, et
dans lequel L'aLP (102) et le vLP (104) sont configurés pour assurer collectivement au moins un fonctionnement VDD, DDI ou VDI, au moins à certains moments lorsqu'au moins un parmi un message a2v transmis par l'aLP (102) n'est pas reçu avec succès par le vLP (104), et/ou un message v2a transmis par le vLP (104) n'est pas reçu avec succès par l'aLP (102), pendant un cycle cardiaque supplémentaire,
le système (100) comprenant un ou plusieurs processeurs (112, 1320), **caractérisé en ce que** les un ou plusieurs processeurs (112, 1320) sont configurés pour :
obtenir le débit a2v, le débit v2a et les informations de séquence d'événements, pendant une période de temps pendant laquelle l'aLP (102) et le vLP (104) ont été configurés pour fournir collectivement l'opération DDD, dans lequel le débit a2v spécifie la fréquence à laquelle les messages a2v transmis par l'aLP (102) ont été reçus avec succès par le vLP (104) pendant la période de temps pendant laquelle l'aLP (102) et le vLP (104) ont été configurés pour fournir collectivement le fonctionnement DDD, et dans lequel le débit v2a spécifie la fréquence à laquelle les messages v2a transmis par le vLP (104) ont été reçus avec succès par l'aLP (102) pendant la période de temps ;
déterminer, sur la base du débit a2v et du débit v2a, une métrique DDD estimée, une métrique VDD estimée, une métrique DDI estimée et une métrique VDI estimée, pour la période de temps ; et
déterminer une métrique de synchronisation auriculo-ventriculaire, AV, pour la période de temps sur la base de la métrique DDD estimée, de la métrique VDD estimée, de la métrique DDI estimée, de la métrique VDI estimée et des informations de séquence d'événements, la métrique de synchronisation AV spécifiant une estimation de la fréquence à laquelle la synchronisation AV a été obtenue pendant la période de temps.

2. Système (100) selon la revendication 1, dans lequel
le système (100) comprend l'aLP (102) et le vLP (104), chacun d'entre eux comprenant un processeur respectif parmi un ou plusieurs processeurs (112, 1320), et chacun d'entre eux comprenant au moins deux électrodes respectives (108) ;
l'aLP (102) est configuré pour être implanté dans ou sur une chambre cardiaque auriculaire ;
le vLP (104) est configuré pour être implanté dans ou sur une chambre cardiaque ventriculaire ; et
l'aLP (102) est configuré pour transmettre des messages a2v au vLP (104), le vLP (104) est configuré pour tenter de recevoir les messages a2v de l'aLP (102), le vLP (104) est configuré pour transmettre des messages v2a à l'aLP (102), et l'aLP (102) est configuré pour tenter de recevoir les messages v2a du vLP (104).

3. Système (100) selon la revendication 2, dans lequel le processeur (112, 1320) du vLP (104) ou le processeur (112, 1320) de l'aLP (102) comprend au moins un des un ou plusieurs processeurs (112, 1320) configurés pour déterminer la métrique de synchronisation AV ; et
au moins l'un parmi le processeur (112, 1320) du vLP (104) ou le processeur (112, 1320) de l'aLP (102) est en outre configuré pour ajuster un ou plusieurs paramètres d'au moins l'un de l'aLP (102) ou du vLP (104) sur la base de la métrique de synchronisation AV.

4. Système (100) selon l'une quelconque des revendications 1 à 3, dans lequel
le système (100) comprend un sous-système non implanté (109) qui comprend au moins un processeur (112, 1320), parmi les un ou plusieurs processeurs (112, 1320), qui est configuré pour déterminer la métrique de synchronisation AV pour la période de temps ; et
les un ou plusieurs processeurs (112, 1320) du sous-système non implanté (109) sont configurés pour ajuster un ou plusieurs paramètres d'au moins l'un de l'aLP (102) ou du vLP (104) sur la base de la métrique de synchronisation AV.

5. Système selon la revendication 4, dans lequel l'aLP (102) et le vLP (104) sont configurés pour communiquer avec le sous-système non implanté (109) à l'aide d'une communication conduite.

6. Système selon la revendication 4 ou 5, dans lequel le sous-système non implanté (109) comprend un programmateur qui est configuré pour communiquer avec l'aLP (102) et le vLP (104).

7. Système selon l'une quelconque des revendications 4 à 6, dans lequel le sous-système non implanté (109) comprend un dispositif parmi une tablette (1504), un téléphone intelligent (1506) ou une montre intelligente, qui est configuré pour communiquer avec l'aLP (102) et le vLP (104).

8. Système selon l'une quelconque des revendications 4 à 7, dans lequel le sous-système non implanté (109) comprend l'un d'un moniteur de chevet (1508), d'un module de liaison patient ou d'un réseau de soins aux patients à distance.

9. Système (100) selon l'une quelconque des revendications 1 à 8, dans lequel les un ou plusieurs processeurs (112, 1320) qui sont configurés pour déterminer la métrique de synchronisation AV pour la période de temps, sont également configurés pour ajuster, sur la base de la métrique de synchronisation AV, au moins un paramètre utilisé par l'aLP (102) pour transmettre d'autres messages a2v au vLP (104), au moins un paramètre utilisé par le vLP (104) pour recevoir les autres messages a2v de l'aLP (102), au moins un paramètre utilisé par le vLP (104) pour transmettre d'autres messages v2a à l'aLP (102), et/ou au moins un paramètre utilisé par l'aLP (102) pour recevoir les autres messages v2a du vLP (104).

10. Système (100) selon l'une quelconque des revendications 1 à 9, dans lequel les un ou plusieurs processeurs (112, 1320) qui sont configurés pour déterminer la métrique de synchronisation AV pour la période de temps, sont également configurés pour provoquer l'affichage de la métrique de synchronisation AV.

11. Système (100) selon l'une quelconque des revendications 1 à 10, dans lequel les un ou plusieurs processeurs (112, 1320) qui sont configurés pour déterminer la métrique de synchronisation AV pour la période de temps, sont également configurés pour fournir une notification indiquant qu'un emplacement d'implant d'au moins l'un de l'aLP (102) ou du vLP (104) doit être modifié.

12. Système (100) selon l'une quelconque des revendications 1 à 11, dans lequel la métrique DDD estimée spécifie une estimation de la fréquence à laquelle l'aLP (102) et le vLP (104) ont fourni collectivement le fonctionnement DDD pendant la période de temps pendant laquelle l'aLP (102) et le vLP (104) ont été configurés pour fournir collectivement le fonctionnement DDD ;
la métrique VDD estimée spécifie une estimation de la fréquence à laquelle l'aLP (102) et le vLP (104) ont fourni collectivement le fonctionnement VDD pendant la période de temps ;
la métrique DDI estimée spécifie une estimation de la fréquence à laquelle l'aLP (102) et le vLP (104) ont fourni collectivement le fonctionnement DDI pendant la période de temps ;
et la métrique VDI estimée spécifie une estimation de la manière dont l'aLP (102) et le VLP (104) ont collectivement fourni le fonctionnement VDI pendant la période de temps.

13. Système (100) selon l'une quelconque des revendications 1 à 12, dans lequel
les informations de séquence d'événements incluent les informations AP-VP, les informations AP-VS, les informations AS-VP et les informations AS-VS ;
les informations AP-VP spécifient la fréquence à laquelle l'aLP (102) a fourni une stimulation auriculaire, AP, et le vLP (104) a fourni une stimulation ventriculaire, VP, pour un même cycle cardiaque au cours de la période de temps pendant laquelle l'aLP (102) et le vLP (104) ont été configurés pour fournir collectivement le fonctionnement DDD ;
les informations AP-VS spécifient la fréquence à laquelle l'aLP (102) a fourni l'AP et le vLP (104) a fourni la détection ventriculaire, VS, pour un même cycle cardiaque au cours de la période de temps ;
les informations AS-VP spécifient la fréquence à laquelle l'aLP (102) a fourni une détection auriculaire, AS, et le vLP (104) a fourni une VP pour un même cycle cardiaque au cours de la période de temps ; et
les informations AS-VS précisent la fréquence à laquelle l'aLP (102) a fourni une AS et le vLP (104) a fourni une VS pour un même cycle cardiaque au cours de la période de temps.

14. Système (100) selon l'une quelconque des revendications 1 à 13, dans lequel les un ou plusieurs processeurs (112, 1320) sont configurés pour :
déterminer que la métrique DDD estimée est égale à un minimum du débit a2v et du débit v2a, ou est égale à un maximum de zéro et 100 % moins une somme d'un pourcentage de messages a2v qui n'ont pas été reçus avec succès par le vLP (104) et un pourcentage de messages v2a qui n'ont pas été reçus avec succès par l'aLP (102) :
déterminer que la métrique VDD estimée est égale au débit a2v moins la métrique DDD estimée ;
déterminer que la métrique DDI estimée est égale au débit v2a moins la métrique DDD estimée ; et
déterminer que la métrique VDI estimée est égale à 100 % moins une somme de la métrique DDD estimée, de la métrique VDD estimée et de la métrique DDI estimée.

15. Système selon l'une quelconque des revendications 1 à 14, dans lequel l'aLP (102) et le vLP (104) sont configurés pour communiquer l'un avec l'autre à l'aide d'une communication conduite.
